# EUROPEAN PATENT APPLICATION

(11) **EP 4 245 308 A1**
(43) Date of publication of application: **20.09.2023**
(21) Application number: 21892029.6
(22) Date of filing: 16.11.2021
(51) Int. Cl.: A61K 35/51, A61P 21/00, C12N 5/077

(54) **CELL PREPARATION TO BE USED FOR PREVENTING DECREASE IN MUSCLE MASS**

(30) Priority: 16.11.2020 JP 2020190551
(71) Applicant: Human Life Cord Japan Inc., Tokyo 103-0012 (JP)
(72) Inventor: CHENG Xianwu, Nagoya-shi, Aichi 464-8601 (JP); HUANG Zhe, Nagoya-shi, Aichi 464-8601 (JP); PIAO Limei, Nagoya-shi, Aichi 464-8601 (JP); KUZUYA Masafumi, Nagoya-shi, Aichi 464-8601 (JP); HARATA Masamitsu, Tokyo 103-0012 (JP)
(74) Representative: J A Kemp LLP
(86) International application number: PCT/JP2021/042028
(87) International publication number: WO 2022/102784

(57) **Abstract**

A cell preparation that can suppress muscle mass loss. A cell preparation for use in suppressing muscle mass loss includes: umbilical cord-derived cells.

## Description

### TECHNICAL FIELD

The present invention relates to a cell preparation for use in suppressing muscle mass loss.

### BACKGROUND ART

In Japan, the population is rapidly aging, and the number of elderly people in 2025 is projected to be 30% or more of the total population. Elderly people suffer various life function disorders with aging. As one of the causes of the life function disorders, age-related muscle loss, which is a disease associated with muscle mass loss with aging,) is known. As one of the age-related muscle loss, sarcopenia is known. Sarcopenia is closely related to "light-headedness", "falling", and "frailty" in the elderly people, and it is known that a situation requiring nursing care can follow thereafter, which is a problem (Non Patent Literature 1). So far, improving nutritional status, increasing daily physical activity, and exercising lightly are recommended, but such practices are not easy for many elderly people and there is a demand for new therapeutic interventions.

### Citation List

### Non Patent Literature

Non Patent Literature 1: Masafumi KATSUYA, "3. Diagnosis, Disease, and Treatment of Sarcopenia", 2015, Nippon Ronen Igakkai Zasshi (Japanese Journal of Geriatrics), Vol. 52, No. 4, pp. 343-349

### SUMMARY OF INVENTION

### Technical Problem

As a method for treating age-related muscle loss, an intervention therapy related to exercise or nutrition has been performed. However, it is currently unclear whether either method has a sufficient effect.

With the foregoing in mind, it is an object of the present invention to provide a cell preparation that can suppress muscle mass loss.

### Solution to Problem

In order to achieve the above object, the present invention provides a cell preparation for use in suppressing muscle mass loss, including: umbilical cord-derived cells.

The present invention also provides a cell preparation for use in treatment of age-related muscle loss, including: the cell preparation for use in suppressing muscle mass loss according to the present invention.

### Advantageous Effects of Invention

According to the present invention, muscle mass loss can be suppressed.

### BRIEF DESCRIPTION OF DRAWINGS

[FIG. 1] FIG. 1 shows graphs showing the change in body weight in Example 1.
[FIG. 2] FIG. 2 shows graphs showing the change in grip strength per body weight in Example 1.
[FIG. 3] FIG. 3 shows graphs showing the endurance in Example 1.
[FIG. 4] FIG. 4 shows graphs showing the measurement results of the muscle weight in Example 1.
[FIG. 5] FIG. 5 shows photographs showing the staining results of the muscle tissue in Example 2.
[FIG. 6] FIG. 6 shows graphs showing the expression levels of PGC1-α, COX4, and GLUT4 in the gastrocnemius and soleus muscles of the mice in Example 3.
[FIG. 7] FIG. 7 shows photographs showing the results of the Western blotting in Example 3.
[FIG. 8] FIG. 8 shows photographs and a graph showing the measurement results of the mitochondria in Example 3.
[FIG. 9] FIG. 9 shows photographs and graphs showing the results of the apoptosis analysis in Example 4.
[FIG. 10] FIG. 10 shows graphs showing the expression level of the inflammatory cytokine and photographs showing the muscle tissue in Example 5.
[FIG. 11] FIG. 11 shows graphs showing the expression level of TGF-β1 and photographs showing the muscle tissue in Example 6.
[FIG. 12] FIG. 12 shows graphs and photographs showing the expression levels of CatK, Sirt1, and MHC in Example 7.
[FIG. 13] FIG. 13 shows photographs of the exosome by the electron microscope in Example 8.
[FIG. 14] FIG. 14 shows photographs of the exosome marker by the Western blotting in Example 8.
[FIG. 15] FIG. 15 is a graph showing the distribution of the particle size of the exosome marker in Example 8.
[FIG. 16] FIG. 16 shows photographs showing the C2C12 cells that have taken up the exosomes in Example 8.
[FIG. 17] FIG. 17 shows photographs and a graph showing the apoptosis of the C2C12 cells that have taken up the exosomes in Example 8.

### DESCRIPTION OF EMBODIMENTS

### <Preparation for Supprresing Muscle Mass Loss>

As described above, the present invention provides a cell preparation for use in suppressing muscle mass loss (hereinafter, also referred to as a "preparation for suppressing muscle mass loss"), including umbilical cord-derived cells. The preparation for suppressing muscle mass loss of the present invention is characterized in that it includes umbilical cord-derived cells, and other configurations and conditions are not particularly limited. It is presumed that the umbilical cord-derived cell has a function of improving a mitochondrial function, suppressing inflammation, suppressing apoptosis, promoting proliferation of muscle cells, suppressing a muscle disorder, suppressing interstitial fibrosis in muscle tissue, and/or promoting muscle repair in muscle, muscle tissue, or muscle cells (also referred to as "muscle fibers" or "muscle cells", hereinafter the same applies) as described below. Therefore, the preparation for suppressing muscle mass loss of the present invention can suppress the muscle mass loss by including the umbilical cord-derived cells.

As used herein, the term "muscle mass" means, for example, the muscle mass of the extremities in a subject, and specifically means the amount of the gastrocnemius muscle, for example. In the present invention, the term "muscle mass" may be evaluated, for example, by measuring the muscle weight of the subject to be measured, or may be evaluated by indirectly calculating the volume using images obtained using dual-energy X-ray absorptiometry (DEXA), bioelectrical impedance, computed tomography (CT), or nuclear magnetic resonance imaging (MRI). When the subject to be measured is a mammal other than a human, the muscle mass is preferably evaluated by measuring the weight of the muscle of the subject to be measured. On the other hand, when the subject to be measured is a human, the muscle mass is preferably evaluated by indirectly calculating the volume using a dual-energy X-ray absorptiometry (DEXA), a bioelectrical impedance method, or the like.

As used herein, the term "suppressing muscle mass loss" means that the muscle mass loss (also referred to as "decrease", "regression", or "reduction", hereinafter, the same applies) is significantly suppressed (also referred to as "inhibited", "repressed", or "prevented", hereinafter, the same applies). Specifically, the "suppressing muscle mass loss" means, for example, that a subject to whom the cell preparation is administered significantly suppresses the degree of muscle mass loss as compared with a subject to whom the cell preparation is not administered. Therefore, in the present invention, it can be said that, "suppressing muscle mass loss" is achieved in the subject to which the cell preparation is administered if the degree of muscle mass loss is significantly suppressed as compared with the subject to which the cell preparation is not administered, even if the muscle mass is decreased as compared with the start of administration of the cell preparation.

It is presumed that the cell preparation of the present invention exhibits an effect of suppressing muscle mass loss by increasing muscle mass, as described in the following Examples. Thus, the cell preparation for use in suppressing muscle mass loss of the present invention can also be referred to as a cell preparation for use in increasing (also referred to as "enhancing", "potentiating", or "expanding", hereinafter, the same applies) muscle mass, for example.

As described in the following Examples, the cell preparation of the present invention suppresses a decrease in the muscle strength such as the sustained muscle strength or promotes an increase in the muscle strength. This is presumed to be because the cell preparation of the present invention exhibits a function of suppressing muscle mass loss or the like. For this reason, the cell preparation for use in suppressing muscle mass loss of the present invention can also be referred to as, for example, a cell preparation for use in suppressing or increasing (hereinafter, also referred to as "enhancing" or "potentiating") muscle strength.

As used herein, the term "umbilical cord" means a white tubular tissue that connects the fetus and the placenta and does not include the placenta and umbilical cord blood. In the present invention, the origin of the "umbilical cord" is not particularly limited, and the "umbilical cord" is derived, for example, from mammals such as mice, rats, rabbits, dogs, cats, cows, horses, pigs, monkeys, dolphins, and sea lions, is preferably derived from primate mammals, and is more preferably derived from humans.

In the present invention, the "umbilical cord" may be an umbilical cord collected from a subject to be administered, treated, or cared (hereinafter, referred to as "subject to be administered"), or may be an umbilical cord collected from a subject other than the subject to be administered. From the viewpoint of not being limited during preparation, it is desirable to use an umbilical cord collected from a subject other than the subject to be administered. The umbilical cord-derived cell of the present invention may be an umbilical cord-derived cell collected from a subject other than the subject to be administered, as described in Examples described below. It has been confirmed that the umbilical cord-derived cells have, for example, a therapeutic effect without being eliminated by immune rejection.

In the present invention, the umbilical cord can be recovered by appropriately removing the placenta from the puerperal tissue including the placenta and/or umbilical cord delivered by, for example, vaginal delivery or caesarean section. In the present invention, the umbilical cord may be obtained by removing the umbilical cord blood from the recovered umbilical cord, or may be further subjecting to a sterilization treatment or a bacteriostasis treatment. The umbilical cord can be removed, for example, by rinsing or perfusing with a solution containing an anticoagulant such as heparin. The sterilization treatment or bacteriostasis treatment is not particularly limited, and may be performed, for example, by application of a disinfectant such as popidone-iodo; immersion in a medium or a buffer to which an antibiotic such as penicillin, streptomycin, amphotericin B, gentamicin, and/or nystatin, and/or an antifungal agent are added; and the like. The umbilical cord may selectively lyse red blood cells, for example, as needed. As a method for selectively lysing the red blood cells, for example, a method known in the art such as incubation in a hypertonic medium or a hypotonic medium by lysis with ammonium chloride can be used.

As used herein, the term "umbilical cord-derived cell" means a cell population prepared using the umbilical cord as a raw material.

The umbilical cord-derived cell of the present invention may be, for example, a cell population having any one or more of the following characteristics (a) to (c), and is preferably a cell population having all of the characteristics:
(a) being adhesive to plastics in culture in the presence of medium;
(b) positive for CD105, CD73, CD90, CD44, HLA-class I, HLA-G5, and programmed cell death 1 ligand (PD-L) 2, and negative for CD45, CD34, CD11b, CD19 and HLA-Class II; and
(c) inducing expression of a gene and/or a protein of indoleamine 2,3-dioxygenase (IDO), prostaglandin E2 (PGE₂), or PD-L1 under an inflammatory condition.

As used herein, the term "positive" means that a signal or the like higher than that of a negative control cell that does not express the antigen or a negative control reaction using an antibody that does not react with the antigen is detected by an analysis method such as flow cytometry detected utilizing an antigen-antibody reaction. In addition, as used herein, the term "negative" means that a signal or the like equivalent to or lower than that of a negative control cell that does not express the antigen or a negative control reaction using an antibody that does not react with the antigen is detected.

As used herein, the term "HLA-class I" means HLA-A, B, or C. As used herein, the term "HLA-Class II" means HLA-DR, DQ, or DP.

In the present invention, the expression "under conditions of inflammation" refers to a condition to be contacted with or to be supplemented with an inflammatory cytokine such as interferon-γ.

In the present invention, the umbilical cord-derived cell may be an extract and/or secretion of the umbilical cord-derived cell. Examples of the extract of the umbilical cord-derived cell include products obtained by subjecting the umbilical cord-derived cells to condensation treatment, centrifugation treatment, drying treatment, freeze-drying treatment, solvent treatment, surfactant treatment, enzyme treatment using protease, glycolytic enzyme, or the like, protein extraction treatment, ultrasonic treatment, and/or grinding treatment; and products obtained by subjecting the umbilical cord-derived cells to combinations of these treatments. Examples of the secretion of the umbilical cord-derived cell include exosomes (extracellular vesicles) and cell culture supernatants of umbilical cord-derived cell.

As used herein, the term "extracellular vesicle" means a vesicle having a membrane secreted from a cell. It is believed that the extracellular vesicles are generally formed within the endosomes of the cells of origin and then released out of the cells. Therefore, the extracellular vesicle usually includes a lipid bilayer membrane and a lumen, wherein the lumen is surrounded by the lipid bilayer membrane. In addition, the lipid bilayer membrane includes lipids derived from the cell membrane of the cell of origin. The lumen includes a cytoplasm derived from a cell of origin. The extracellular vesicles are classified into, for example, exosomes, micro vesicles (MVs), apoptotic bodies, and the like according to their size and/or surface marker.

The mean diameter (weighted average) of the extracellular vesicles is, for example, from 1 to 500 nm, preferably from 10 to 400 nm, more preferably from 30 to 400 nm. The mean diameter can be measured according to Example 8 described below. The mean diameter of the extracellular vesicles can be adjusted by, for example, filtering a liquid containing the extracellular vesicles using a filter having a desired pore diameter or the like.

In the present invention, it is preferable that the cell preparation exhibits an effect of suppressing interstitial fibrosis in muscle tissue. The effect of suppressing interstitial fibrosis can be evaluated, for example, by using the fibrosis area in the interstitium of the muscle tissue as an indicator according to Example 2 to be described below, and specifically, by using the fibrosis area of the interstitium in muscle tissue (e.g., gastrocnemius muscle or soleus muscle) of the SAMP10 mouse as an indicator (interstitial fibrosis assay). In the evaluation, for example, when the fibrotic area of the interstitium in the test substance-administration group is 10% or less, 15% or less, 20% or less, 25% or less, 30% or less, 35% or less, 40% or less, 45% or less, 50% or less, 55% or less, 60% or less, 65% or less, 70% or less, 75% or less, 80% or less, 85% or less, 90% or less, 95% or less, 96% or less, 97% or less, 98% or less, or 99% or less, as compared with the fibrotic area of the interstitium in the test substance-non-administration group, the test substance can be evaluated as having an effect of suppressing interstitial fibrosis in muscle tissue. For example, in the interstitial fibrosis assay, the cell preparation can reduce the interstitial fibrosis to 10% or less, 15% or less, 20% or less, 25% or less, 30% or less, 35% or less, 40% or less, 45% or less, 50% or less, 55% or less, 60% or less, 65% or less, 70% or less, 75% or less, 80% or less, 85% or less, 90% or less, 95% or less, 96% or less, 97% or less, 98% or less, or 99% or less, as compared with the cell preparation-non-administration group. Since the preparation for suppressing muscle mass loss of the present invention can suppress interstitial fibrosis in muscle tissue, it can also be referred to as, for example, a cell preparation for use in suppressing interstitial fibrosis in muscle tissue.

As used herein, the term "muscle tissue" means a tissue composed of muscle cells (muscle fibers), and is, for example, an excitable tissue having contractility.

As used herein, the term "interstitium" means a space between muscle fibers.

In the present invention, it is preferable that the cell preparation exhibits an effect of improving a mitochondrial function. The mitochondrial function-improving effect can be evaluated using, for example, the expression level of the mitochondrial function-improving gene such as peroxisome proliferator-activated receptor gamma coactivator 1-α (PGC1-α), cytochrome c oxidase subunit 4 (COX4), glucose transporter type 4 (GLUT4), or the like in muscle tissue or the number of mitochondria in muscle tissue as an indicator according to Example 3 to be described below, and specifically, the mitochondrial function-improving effect can be evaluated using the expression level of the mitochondrial function-improving gene in mitochondria in muscle tissue (e.g., gastrocnemius muscle or soleus muscle) of the SAMP10 mouse or the number of mitochondria in muscle tissue (e.g., gastrochondrial muscle or soleus muscle) as an indicator (mitochondrial function assay). In the evaluation, for example, when the expression level of the mitochondrial function-improving gene in the test substance-administration group is 1.5 times or more, 2 times or more, 2.5 times or more, 5 times or more, 10 times or more, 25 times or more, 50 times or more, 100 times or more, 125 times or more, or 250 times or more as compared with the expression level of the mitochondrial function-improving gene in the test substance-non-administration group, the test substance can be evaluated as having an effect of improving a mitochondrial function. Further, in the evaluation, for example, when the number of mitochondria in the test substance-administration group is 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 100% or more, as compared with the number of mitochondria in the test substance-non-administration group, the test substance can be evaluated as having an effect of improving a mitochondrial function. For example, in the mitochondrial function assay, the cell preparation can increase the expression level of the mitochondrial function-improving gene by 1.5 times or more, 2 times or more, 2.5 times or more, 5 times or more, 10 times or more, 25 times or more, 50 times or more, 100 times or more, 125 times or more, or 250 times or more, as compared with the cell preparation-non-administration group, and the upper limit thereof is, for example, 1000 times or less or 500 times or less. For example, in the mitochondrial function assay, the cell preparation can increase the number of mitochondria by 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 100% or more, as compared with the cell preparation-non-administration group, and the upper limit thereof is, for example, 150% or less or 125% or less. Since the mitochondrial function is improved in muscle tissue, the preparation for suppressing muscle mass loss of the present invention can also be referred to, for example, as a cell preparation for use in improving a mitochondrial function in muscle tissue. In addition, since the preparation for suppressing muscle mass loss of the present invention can induce expression of a gene related to a mitochondrial function in muscle tissue, it can also be referred to as, for example, a cell preparation for use in inducting (expression inducing) a mitochondrial function-improving gene in muscle tissue. In addition, since the preparation for suppressing muscle mass loss of the present invention can increase the number of mitochondria in muscle tissue, it can also be referred to as, for example, a cell preparation for use in increasing the number of mitochondria in muscle tissue.

Regarding the base sequences of the mRNA of the human PGC1-α, the human COX4, and the human GLUT4 and the amino acid sequences of the proteins of the human PGC1-α, the human COX4, and the human GLUT4, for example, reference can be made to the sequences registered under the following Genbank accession numbers.
- Human PGC1-α
   mRNAAccession Number: NM_001330751
   Protein Accession Number: NP_001317680
- Human COX4
   mRNAAccession Number: NM_001861
   Protein Accession Number: NP_001852
- Human GLUT4
   mRNAAccession Number: NM_001042
   Protein Accession Number: NP_001033

In the present invention, it is preferable that the cell preparation exhibit an effect of suppressing apoptosis of muscle cells. The effect of suppressing apoptosis of muscle cells can be evaluated, for example, by using the expression level of Cleaved-caspase-3 and/or Cleaved-caspase-8 or the fragmentation of DNA (fragmentation of genomic DNA) as an indicator according to Example 4 to be described below, and specifically, by using the number of apoptotic cells in muscle tissue (e.g., gastrocnemius muscle or soleus muscle) of the SAMP10 mouse as an indicator (apoptosis assay). In the evaluation, for example, when the number of apoptotic cells in the test substance-administration group is 10% or less, 15% or less, 20% or less, 25% or less, 30% or less, 35% or less, 40% or less, 45% or less, 50% or less, 55% or less, 60% or less, 65% or less, 70% or less, 75% or less, 80% or less, 85% or less, 90% or less, 95% or less, 96% or less, 97% or less, 98% or less, or 99% or less, as compared with the number of apoptotic cells in the test substance-non-administration group, the test substance can be evaluated as having an effect of suppressing apoptosis of muscle cells. For example, in the apoptosis assay, the cell preparation can reduce the number of apoptotic cells to 10% or less, 15% or less, 20% or less, 25% or less, 30% or less, 35% or less, 40% or less, 45% or less, 50% or less, 55% or less, 60% or less, 65% or less, 70% or less, 75% or less, 80% or less, 85% or less, 90% or less, 95% or less, 96% or less, 97% or less, 98% or less, or 99% or less, as compared with the cell preparation-non-administration group. Since the preparation for suppressing muscle mass loss of the present invention shows suppression of apoptosis of muscle cells, for example, it can also be referred to as a cell preparation for use in suppressing apoptosis of muscle cells. The muscle cell is, for example, a skeletal muscle cell.

In the present invention, it is preferable that the cell preparation exhibit an anti-inflammatory effect. The anti-inflammatory effect can be evaluated, for example, by using the inducibility of an inflammatory cytokine gene or a chemokine gene as an indicator according to Example 5 to be described below, and specifically, by using the expression level of an inflammatory cytokine gene or a chemokine gene in muscle tissue (e.g., gastrocnemius muscle or soleus muscle) of the SAMP 10 mouse as an indicator (inflammatory assay). In the evaluation, for example, when the expression level of an inflammatory cytokine gene or a chemokine gene in the test substance-administration group is 10% or less, 15% or less, 20% or less, 25% or less, 30% or less, 35% or less, 40% or less, 45% or less, 50% or less, 55% or less, 60% or less, 65% or less, 70% or less, 75% or less, 80% or less, 85% or less, 90% or less, 95% or less, 96% or less, 97% or less, 98% or less, or 99% or less, as compared with the expression level of the inflammatory cytokine gene or the chemokine gene in the test substance-non-administration group, the test substance can be evaluated as having an anti-inflammatory effect.
For example, in the inflammation assay, the cell preparation can reduce the expression level of an inflammatory cytokine gene and/or a chemokine gene to 10% or less, 15% or less. 20% or less, 25% or less, 30% or less, 35% or less, 40% or less, 45% or less, 50% or less, 55% or less, 60% or less, 65% or less, 70% or less, 75% or less, 80% or less, 85% or less, 90% or less, 95% or less, 96% or less, 97% or less, 98% or less, or 99% or less, as compared with the cell preparation-non-administration group. Since the preparation for suppressing muscle mass loss of the present invention exhibits an anti-inflammatory effect in muscle tissue, it can also be referred to as a cell preparation for use in suppressing inflammation in muscle tissue, for example. In addition, since the preparation for suppressing muscle mass loss of the present invention can suppress the induction of the expression of an inflammatory cytokine gene such as a Tumor Necrosis Factor (TNF)-α and/or a chemokine gene such as MCP-1 (CCL2, monocyte chemotactic and activating factor) in muscle tissue, it can also be referred to as, for example, a cell preparation for use in suppressing expression or suppressing induction of expression of an inflammatory cytokine gene and/or a chemokine gene in muscle tissue.

Regarding the base sequences of the mRNA of the human TNF-α and the human MCP-1 and the amino acid sequences of the proteins of the human TNF-α and the human MCP-1, for example, reference can be made to the sequences registered under the following Genbank accession numbers.
- Human TNF-α:
   mRNAAccession Number: NM_000594
   Protein Accession Number: NP_000585
- Human MCP-1
   mRNAAccession Number: NM_002982
   Protein Accession Number: NP_002973

In the present invention, it is preferable that the cell preparation exhibit an effect of promoting proliferation of muscle cells. The proliferation-promoting effect can be evaluated, for example, by using the proliferation of muscle cells or the expression of a muscle cell proliferation-inducing gene such as a transforming growth factor (TGF)-β1 as an indicator according to Example 6 to be described below, and specifically, by using the proliferation of muscle cells or the expression level of a muscle cell proliferation-inducing gene in muscle tissue (e.g., gastrocnemius muscle or soleus muscle) of the SAMP 10 mouse as an indicator (muscle proliferation assay). In the evaluation, for example, when the number of muscle cells in the test substance-administration group is 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, 96% or more, 97% or more, 98% or more, or 99% or more, as compared with the number of muscle cells in test substance-non-administration group, the test substance can be evaluated as having an effect of promoting proliferation of muscle cells. In addition, in the evaluation, for example, when the expression level of the muscle cell proliferation-inducing gene in the test substance-administration group is 1.5 times or more, 2 times or more, 2.5 times or more, 5 times or more, 10 times or more, 25 times or more, 50 times or more, 100 times or more, 125 times or more, 250 times or more, or 500 times or more, as compared with the expression level of the muscle cell proliferation-inducing gene in the test substance-non-administration group, the test substance can be evaluated as having an effect of promoting proliferation of muscle cells. For example, in the muscle proliferation assay, the cell preparation can increase the number of muscle cells by 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, 96% or more, 97% or more, 98% or more, or 99% or more, as compared with the cell preparation-non-administration group, and the upper limit thereof is, for example, 150% or less or 100% or less. For example, in the muscle proliferation assay, the cell preparation can increase the expression level of the muscle cell proliferation-inducing gene by 1.5 times or more, 2 times or more, 2.5 times or more, 5 times or more, 10 times or more, 25 times or more, 50 times or more, 100 times or more, 125 times or more, 250 times or more, or 500 times or more, as compared with the cell preparation-non-administration group, and the upper limit thereof is, for example, 1000 times or less or 750 times or less. Since the preparation for suppressing muscle mass loss of the present invention can promote (also referred to as "accelerate", "increase", or "potentiate", hereinafter, the same applies) the proliferation of the muscle cells, it can also be referred to as, for example, a cell preparation for use in promoting the proliferation of the skeletal muscle of muscle cell or muscle tissue. In addition, since the preparation for suppressing muscle mass loss of the present invention can induce the expression of the proliferation-inducing gene of the muscle cell, it can also be referred to as a cell preparation for use in, for example, induction (expression induction) of the proliferation-promoting gene in muscle tissue. The muscle cell is, for example, a skeletal muscle cell.

Regarding the base sequence of the mRNA of the human TGF-β1 and the amino acid sequence of the protein of the human TGF-β1, for example, reference can be made to the sequences registered under the following Genbank accession number.
- Human TGF-β1:
   mRNAAccession Number: NM_000660
   Protein Accession Number: NP_000651

In the present invention, it is preferable that the cell preparation promotes the repair of muscle tissue, preferably the repair of skeletal muscle fibers. The repair ability of muscle tissue can be evaluated, for example, by using the expression level of a repair-promoting gene such as a Sirt 1 (Sirtuin 1) gene, a myosin heavy chain (MHC) gene, or the like in muscle tissue, or the expression level of a protein of the repair-promoting gene such as a Sirt 1 and/or a myosin heavy chain as an indicator according to Example 7 to be described below, and specifically, by using the expression level of the repair-promoting gene in muscle tissue (e.g., gastrocnemius muscle or soleus muscle) of the SAMP 10 mouse (muscle repair assay). In the evaluation, for example, when the expression level of the repair-promoting gene in muscle tissue in the test substance-administration group is 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, 96% or more, 97% or more, 98% or more, or 99% or more, as compared with the expression level of the repair-promoting gene in muscle tissue in the cell preparation-non-administration group, the test substance can be evaluated as having a muscle tissue-repairing effect. For example, in the muscle repairing assay, the cell preparation can increase the expression level of the repair-promoting gene in muscle tissue by 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, 96% or more, 97% or more, 98% or more, or 99% or more, as compared with the cell preparation-non-administration group, and the upper limit thereof is, for example, 150% or less or 100% or less. Since the preparation for suppressing muscle mass loss of the present invention exhibits the repair ability of muscle tissue or skeletal muscle, it can also be referred to as, for example, a cell preparation for use in the repair of muscle tissue or skeletal muscle. In addition, since the preparation for suppressing muscle mass loss of the present invention can induce the expression of the repair-promoting gene or the expression of the protein of the repair-promoting gene in muscle tissue or skeletal muscle, it can also be referred to as a cell preparation for use in promoting the repair of muscle tissue or a cell preparation for use in inducing the expression of the repair-promoting gene in muscle tissue. The muscle tissue is, for example, a muscle tissue of skeletal muscle.

Regarding the base sequences of the mRNA of the human SIRT1 and the human MHC and the amino acid sequences of the proteins of the human SIRT1 and the human MHC, for example, reference can be made to the sequence registered under the following Genbank accession number.
- Human SIRT1:
   mRNA Accession Number: NM_012238
   Protein Accession Number: NP_036370
- Human MHC:
   mRNAAccession Number: NM_005963
   Protein Accession Number: NP_005954

In the present invention, it is preferable that the cell preparation suppress disorder of muscle tissue, preferably suppresses disorder of muscle tissue of skeletal muscle. The muscle tissue disorder suppressing ability can be evaluated, for example, by using the expression level of a muscle tissue disorder-inducing gene such as a cathepsin K (CatK, CTSK) gene as an indicator according to Example 7 to be described below. The CatK causes a muscle disorder, for example, by inducing degradation of muscle cells. Since the preparation for suppressing muscle mass loss of the present invention exhibits the ability to suppress disorder of muscle tissue or muscle tissue of skeleton muscle, it can also be referred to as, for example, a cell preparation for use in suppressing disorder of muscle tissue or muscle tissue of skeletal muscle. In addition, since the preparation for suppressing muscle mass loss of the present invention can suppress the expression of the muscle disorder-inducing gene in muscle tissue or skeletal muscle, it can also be referred to as a cell preparation for use in suppressing the expression of the muscle disorder-inducing gene. The muscle tissue is, for example, muscle tissue of skeletal muscle.

Regarding the base sequence of the mRNA of the human CatK and the amino acid sequence of the protein of the human CatK, for example, reference can be made to the sequence registered under the following Genbank accession number.
- Human CatK:
   mRNA Accession Number: NM_000396
   Protein Accession Number: NP_000387

In the present invention, a method for producing (preparing) the umbilical cord-derived cell may include, for example, a step of isolating cells from the umbilical cord, and optionally, a step of passaging the isolated cells. As a specific example, the preparation method includes, for example, the steps of (1) cutting the umbilical cord, (2) culturing the umbilical cord section, and (3) passaging. As another example, the preparation method includes the steps of (A) cutting or enzymatically treating the umbilical cord or dissociating the tissue by both of the cutting and enzymatically treating (B) culturing the umbilical cord tissue, and (C) passaging. The umbilical cord-derived cells may be a uniform cell population or a heterogeneous cell population.

When the cord-derived cells are prepared by the method including the steps (1) to (3), or the method including the steps (A) to (C), for example, the method for preparing umbilical cord-derived cells can be performed as follows. The method for preparing umbilical cord-derived cells, however, is not limited to the following examples.

First, the method including the steps (1) to (3) will be described. The step (1) of cutting the umbilical cord may be performed, for example, by cutting the umbilical cord obtained by the above-described method by mechanical force (shredding force or shearing force) in a state of including amnion, blood vessels, perivascular tissue and/or Walt Jelly. The size of the umbilical cord section obtained by cutting is not particularly limited, and is, for example, 1 to 10 mm³, 1 to 5 mm³, 1 to 4 mm³, 1 to 3 mm³ or 1 to 2 mm³.

Second, in the step (2) of culturing the umbilical cord section, for example, the umbilical cord section obtained by cutting is seeded into an incubator such as a petri dish, a dish, or a flask, and is cultured in a culture solution suitable for umbilical cord-derived cells. In the step (2), it is preferable that the umbilical cord section be not subjected to digestive enzyme treatment.

In the present invention, the "incubator" may be, for example, an incubator having a solid surface. As the incubator, for example, an incubator for use in culturing cells, tissues, and/or organs can be used. The "solid surface" means, for example, any material capable of binding to the umbilical cord-derived cells. Specifically, the material may be, for example, a plastic material that has been subjected to treatment (e.g., hydrophilicity-increasing treatment) to promote binding of mammalian cells to its surface. The type of the incubator having the solid surface is not particularly limited, and examples thereof include a petri dish, a dish, and a flask.

In the present invention, the "culture solution suitable for umbilical cord-derived cells" can be prepared, for example, by adding an additive such as serum to a basal medium. The additive may be, for example, serum and/or one or more serum substitutes such as albumin, transferrin, fatty acids, insulin, sodium selenite, cholesterol, collagen precursors, trace elements, 2-mercaptoethanol, 3 '-thiolglycerol, and the like. The culture solution may further contain substances such as lipids, amino acids, proteins, polysaccharides, vitamins, growth factors, low-molecular-weight compounds, antibiotics, antifungal agents, antioxidants, pyruvic acid, buffers, inorganic salts, and the like, if necessary. The basal medium is not particularly limited, and examples thereof include Dulbecco's Modified Eagle's Medium (DMEM) (high glucose or low glucose), modified DMEM, DMEM/MCDB 201, Eagle's minimal essential medium, Ham's F 10 medium (F10), Ham's F-12 medium (F12), Iscove's modified Dulbecco (IMDM) medium, Fischer's medium, mesenchymal stem cell growth medium (MSCGM), DMEM/F12, RPMI 1640, CELL-GRO-FREE, and mixed media thereof. Examples of the serum include human serum, fetal bovine serum (FBS), bovine serum, calf serum, goat serum, horse serum, pig serum, sheep serum, rabbit serum, and rat serum. The amount of serum added to the basal medium is, for example, 5 v/v% to 15 v/v%, and preferably about 10 v/v%. The fatty acid is not particularly limited, and examples thereof include linol acid, oleic acid, linoleic acid, arachidonic acid, myristic acid, palmitoylic acid, palmitic acid, and stearic acid. The lipid is not particularly limited, and examples thereof include phosphatidylserine, phosphatidylethanolamine, and phosphatidylcholine. The amino acid is not particularly limited, and examples thereof include L forms of amino acids such as L-alanine, L-arginine, L-aspartic acid, L-asparagine, L-cysteine, L-cystine, L-glutamic acid, L-glutamine, L-glycine, and the like; D forms thereof; or mixtures thereof (DL forms) , and the like. The protein is not particularly limited, and examples thereof include ecotine, reduced glutathione, fibronectin, and β2-microglobulin. The polysaccharide is not particularly limited, and examples thereof include glycosaminoglycans such as hyaluronic acid and heparan sulfate. The growth factor is not particularly limited, and examples thereof include platelet-derived growth factor (PDGF), epidermal growth factor (EGF), fibroblast growth factor (FGF), vascular endothelial growth factor (VEGF), insulin-like growth factor-1(IGF-1), leukocyte inhibitory factor (LIF), basic fibroblast growth factor (bFGF), transforming growth factor beta (TGF-β), hepatocyte growth factor (HGF), connective tissue growth factor (CTGF), and erythropoietin (EPO). The antibiotic and/or antifungal agent is not particularly limited, and examples thereof include penicillin G, streptomycin sulfate, amphotericin B, gentamicin, nystatin, and mixtures thereof.

In the step (2), in order to prevent the seeded umbilical cord section from being suspended in the culture solution, it is preferable to hold down the umbilical cord section using a plate or the like during the culture period. The plate may be, for example, the plate described in JP 2015-70824A, which is herein incorporated by reference.

In the step (2), the culture conditions are not particularly limited, and for example, reference can be made to general culture conditions of cells, tissues, organs, and the like. Specifically, the CO₂ concentration in the step (2) is, for example, 0 to 5%. The O₂ concentration in the step (2) is, for example, 2 to 25%, and preferably 5 to 20%. The culture temperature in the step (2) is, for example, 25 to 40°C, and preferably about 37°C (35 to 39°C).

In the step (2), the culture period is not particularly limited, and for example, it is preferable to perform culturing until the cells migrate from the umbilical cord section and the cells become confluent with respect to the incubator by 50%, 60%, 70%, 80% or more.

Then, in the step (2), for example, after the culturing, in order to remove the unbound cells and cellular debris, the cells are washed, and the cells are peeled off by a peeling agent containing solutions containing a chelating agent such as EDTA or the like; proteases such as trypsin, collagenase, dispase, or the like; glycolytic enzyme such as hyaluronidase or the like; or a mixture thereof. In the step (2), for example, the cells and the peel solution containing the umbilical cord section are filtered using a cell strainer or the like, so that only the cells can be obtained as umbilical cord-derived cells. The obtained umbilical cord-derived cells can be seeded into the incubator and cultured using the culture solution, for example.

In the step (3), the umbilical cord-derived cells can be proliferated up to the required number by passage culture. In the step (3), in the passage culture, the culture may be continued by peeling off the cells with the peeling agent and seeding the cells with an appropriate cell density in a separately prepared incubator. When the cells are seeded, the cell density (seeding density) is, for example, 1×10² to 1×10⁵ cells/cm², 5×10² to 5×10⁴ cells/cm², 1×10³ to 1×10⁴ cells/cm², 2×10³ to 1×10⁴ cells/cm², or the like, and is preferably 2×10³ to 1×10⁴ cells/cm². The seeding density is preferably adjusted so that, for example, a period of time until an appropriate confluency is achieved is 3 to 7 days. In the passage culture in the step (3), the medium may be replaced as necessary.

The number of passages in the step (3) is not particularly limited, and may be performed, for example, until senescence when cell division is stopped. The number of passages in the step (3) is preferably 3 to 25 times, and is more preferably 4 to 12 times, from the viewpoint of use in therapy, for example.

Next, a method including the steps (A) to (C) will be described. In the step (A) of enzymatically treating, the umbilical cord obtained by the above-described method may be subjected to an enzyme treatment in a state of including amniotic membrane, blood vessels, perivascular tissue, and/or Walt Jelly to dissociate the tissue. The enzyme used in the enzyme treatment is not particularly limited, and examples thereof include proteases such as collagenase, dispase, and the like; and glycolytic enzymes such as hyaluronidase, and the like.

The step (B) of culturing the umbilical cord tissue and the step (C) of passaging can be performed, for example, in the same manner as the step of (2) culturing the umbilical cord tissue and the step of (3) passaging, respectively.

Thus, after the step (3) or (C), the umbilical cord-derived cells can be obtained.

In order to examine that the cells obtained by the method for preparing umbilical cord-derived cells are the umbilical cord-derived cells, analysis may be performed by a conventional method using flow cytometry or the like with respect to surface antigens or the like. Further, the cells obtained by the method for preparing umbilical cord-derived cells may be evaluated whether they are the umbilical cord-derived cells by measuring the amount of various proteins produced by the cells.

The cells obtained by the method for preparing umbilical cord-derived cells may be prepared for therapeutic use as they are or may be cryopreserved. The cryopreservation is performed, for example, by suspending the umbilical cord-derived cells in a cryopreservation solution capable of storing umbilical cord-derived cells and storing the cells at -80°C to -180°C. The cryopreservation solution is not particularly limited, and may be, for example, an aqueous solution containing a cryoprotectant and glucose. Examples of the cryoprotectant include dimethyl sulfoxide (hereinafter also referred to as "DMSO"), dextran, glycerol, propylene glycol, and 1-methyl-2-pyrrolidone, and the cryoprotectant is preferably DMSO and/or propylene glycol, and more preferably DMSO. The cryoprotectant is, for example, contained in the cryopreservation solution in an amount of 1 to 15 w/v%, preferably 5 to 15 w/v%, more preferably 5 to 12 w/v%, and still more preferably 8 to 11 w/v%. The cryoprotectant is, for example, contained in the cryopreservation solution in an amount of 1 to 15 v/v%, preferably 5 to 15 v/v%, more preferably 5 to 12 v/v%, and still more preferably 8 to 11 v/v%.

The glucose contained in the cryopreservation solution is, for example, in an amount of 0.5 to 10 w/v%, preferably 1 to 10 w/v%, more preferably 2 to 8 w/v%, and still more preferably 2 to 5 w/v%.

The cryopreservation solution may further contain other components. Examples of the other components include pH adjusters and thickeners. Examples of the pH adjuster include sodium bicarbonate, HEPES, and a phosphate buffer solution. When the phosphate buffer solution is not added to a basic stock solution (BSS), the pH adjuster may be, for example, the one to which sodium chloride having a function of giving a buffering ability around a pH suitable for the umbilical cord-derived cells. As the pH adjuster, a phosphate buffer solution is preferably used. The pH adjuster is preferably used to adjust the pH in the cryopreservation solutions to, for example, about 6.5 to 9, preferably 7 to 8.5. In the present invention, the "phosphate buffer solution" refers to a buffer solution containing, for example, sodium chloride, monosodium phosphate (anhydrous), monopotassium phosphate (anhydrous), disodium phosphate (anhydrous), trisodium phosphate (anhydrous), potassium chloride, and potassium dihydrogen phosphate (anhydrous), and is preferably a buffer solution containing sodium chloride, monosodium phosphate (anhydrous), potassium chloride, or potassium dihydrogen phosphate (anhydrous). The pH adjuster is, for example, contained in the cryopreservation solutions in an amount of 0.01 to 1 w/v%, preferably in an amount of 0.05 to 0.5 w/v%.

The cryopreservation solution may or may not contain a natural animal-derived component. Examples of the natural animal-derived component include the aforementioned serum and basal medium. Preferably, the cryopreservation solution does not contain a natural animal-derived component. In the cryopreservation solution containing no natural animal-derived component, there is no difference in quality among production lots of natural animal-derived components, and the possibility of changing the properties of cells in umbilical cord tissue due to various cytokines, growth factors, hormones, and other components contained in serum can be suppressed. In addition, the influence of components from unknown origins contained in the basal medium can be suppressed. For this reason, the cryopreservation solution containing no natural animal-derived components is very useful, especially in clinical use.

The cryopreservation solution may further contain a thickener. The thickener is not particularly limited, and examples thereof include a thickener that can constitute a cryopreservation solution that can sufficiently store the umbilical cord tissue. Examples of the thickener include carboxymethylcellulose (hereinafter, also referred to as "CMC"), sodium carboxymethylcellulose (hereinafter, also referred to as "CMC-Na"), organic acid polymers, propylene glycol alginate, and sodium alginate. As the thickener, CMC and CMC-Na are preferable, and CMC-Na is particularly preferable. The organic acid polymer is preferably sodium polyacrylate. The thickener is, for example, contained in the cryopreservation solution in an amount of 0.1 to 1 w/v%, preferably in an amount of 0.1 to 0.5 w/v%, more preferably in an amount of 0.2 to 0.4 w/v%.

The cryopreservation solution is preferably an aqueous solution. The osmotic pressure of the cryopreservation solution is, for example, preferably 1000 mOsm or more, and more preferably 1000 to 2700 mOsm in order to maintain the performance as the preservation solution.

The cryopreservation solution is preferably an aqueous solution that contains a thickener, a cryoprotectant, and glucose, and is free of natural animal-derived components. The cryopreservation solution is more preferably an aqueous solution that contains CMC-Na, DMSO, and glucose, and is free of natural animal-derived components. The cryopreservation solution is more preferably an aqueous solution that contains CMC-Na in an amount of 0.1 to 1 w/v%, DMSO in an amount of 1.0 to 15 w/v%, and glucose in an amount of 0.5 to 10 w/v%, and is free of natural animal-derived components.

The cells obtained by the method for preparing umbilical cord-derived cells may be used as a cell preparation for various uses, for example, by mixing with an infusion preparation. When the umbilical cord-derived cells are cryopreserved, the cryopreserved umbilical cord-derived cells may be suspended in the cryopreservation solution and used as a cell preparation for various uses after thawing, or may be mixed with an infusion preparation after thawing to obtain a mixture to be used as a cell preparation for various uses. In the case of mixing with the infusion preparation, the culture solution or the cryopreservation solution in which the umbilical cord-derived cells are suspended may be mixed with the infusion preparation or the like, or the culture solution or the cryopreservation solution may be subjected to centrifugation to separate cells from a solvent and then only the cells may be mixed with the infusion preparation. In the preparation method, for example, in order to avoid complication of the procedure, it is preferable that the frozen cells be not subjected to a step of culturing after thawing, or that the cryopreservation solution in which cells after thawing are suspended is directly mixed with an infusion preparation.

In the present invention, the "infusion preparation" is, for example, a solution such as an infusion solution used in the treatment of human, and specific examples thereof include saline, 5% glucose solution, Ringer's solution, Lactate Ringer's solution, Acetate Ringer's solution, No.1 solution, No.2 solution, No.3 solution, and No. 4 solution.

The preparation for suppressing muscle mass loss of the present invention may be a kit including the infusion preparation in addition to the umbilical cord-derived cells.

The preparation for suppressing muscle mass loss of the present invention may include a pharmaceutically acceptable carrier in addition to or in place of the infusion preparation. Examples of the carrier include suspensions, solubilizer, stabilizers, isotonizing agents, preservatives, adsorption inhibitor, surfactants, diluents, media, pH adjusters, painless agents, buffers, sulfur-containing reducing agents, antioxidants, and the like for administering the cell preparation, and the carrier may be suitably added to the extent that it does not interfere with the effect of the present invention.

The suspension is not particularly limited, and examples thereof include methylcellulose, polysorbate 80, hydroxyethylcellulose, gum arabic, powdered tragacanth, sodium carboxymethylcellulose, and polyoxyethylene sorbitan monolaurate.

The solubilizer is not particularly limited, and examples thereof include polyoxyethylene hydrogenated castor oil, polysorbate 80, nicotinic acid amide, polyoxyethylene sorbitan monolaurate, macrogol, and castor oil fatty acid ethyl ester.

The stabilizer is not particularly limited, and examples thereof include dextran 40, methylcellulose, gelatin, sodium sulfite, and sodium metasulfate.

The isotonizing agent is not particularly limited, and examples thereof include D-mannitol and sorbitol.

The preservative is not particularly limited, and examples thereof include methyl parahydroxybenzoate, ethyl parahydroxybenzoate, sorbic acid, phenol, cresol, and chlorocresol.

The adsorption inhibitor is not particularly limited, and examples thereof include human serum albumin, lecithin, dextran, ethylene oxide propylene oxide copolymer, hydroxypropylcellulose, methyl cellulose, hydrogenated castor oil, and polyethylene glycol.

The sulfur-containing reducing agent is not particularly limited, and examples thereof include N-acetylcysteine, N-acetylhomocysteine, thiochitoic acid, thiodiglycol, thioethanolamine, thioglycerol, thiosorbitol, thioglycolic acid and its salts, sodium thiosulfate, glutathione, and those having a sulfohydril group such as a thioalkanoic acid having 1 to 7 carbon atoms.

The antioxidant is not particularly limited, and examples thereof include elisorbic acid, dibutylhydroxytoluene, butylhydroxyanisole, α-tocopherol, tocopherol acetate, L-ascorbic acid and its salts, L-ascorbic acid palmitate, L-ascorbic acid stearate, sodium hydrogen sulfite, sodium sulfite, triamyl gallate, propyl gallate, and chelating agents such as sodium ethylenediamine 4 acetate (EDTA), sodium pyrophostrate, sodium metaphosphate, and the like.

The preparation for suppressing muscle mass loss of the present invention may appropriately further contain a generally added component such as an inorganic salt such as sodium chloride, potassium chloride, calcium chloride, sodium phosphate, potassium phosphate, sodium bicarbonate, or the like; an organic salt such as sodium citrate, potassium citrate, sodium acetate, or the like; or a sugar such as glucose. Further, as the anticoagulant and/or pH adjuster, for example, an ACD-A solution (composition of Na citrate hydrate citrate hydrate, glucose, and the like) may be added.

The preparation for suppressing muscle mass loss of the present invention may be mixed with, for example, an organic substance such as a biopolymer, or the like; an inorganic substance such as hydroxyapatite, or the like; or a substance for topical administration or the like, and specifically may be mixed with, for example, a collagen matrix, a polylactic acid polymer or copolymer, a polyethylene glycol polymer or copolymer, or a chemical derivative thereof.

The preparation for suppressing muscle mass loss of the present invention may be used, for example, *in vitro* or *in vivo.* The preparation for suppressing muscle mass loss of the present invention can be used, for example, as a research reagent or as a pharmaceutical product. In the latter case, the preparation for suppressing muscle mass loss of the present invention can also be referred to as a cell preparation or a pharmaceutical cell preparation for the treatment of age-related muscle loss.

A subject to which the preparation for suppressing muscle mass loss of the present invention is administered is not particularly limited. When the preparation for suppressing muscle mass loss of the present invention is used *in vivo,* the subject to be administered may be, for example, a human or a non-human animal other than a human. Examples of the non-human animal include mammals such as mice, rats, rabbits, dogs, cats, cows, horses, pigs, monkeys, dolphins, sea lions, birds, and fish. When the preparation for suppressing muscle mass loss of the present invention is used *in vitro,* the subject to be administered may be, for example, a cell, a tissue, an organ, or the like, and the cell may be, for example, a cell collected from a living body or a cultured cell, and the tissue or organ may be, for example, a tissue (living tissue) or an organ collected from a living body. Examples of the cell include muscle cells, induced pluripotent stem cells (iPS cells), and stem cells.

When the preparation for suppressing muscle mass loss of the present invention is used *in vivo,* the subject to be administered is a subject with inflammation in muscle tissue, a subject with induced (also referred to as " potentiated", "increased", "improved", or "facilitated") expression of an inflammatory cytokine gene and/or induced expression of a chemokine gene in muscle tissue, a subject with induced interstitial fibrosis in muscle tissue, a subject with suppressed expression of a repair-promoting gene in muscle, a subject with decreased mitochondrial function in muscle cells, a subject with decreased number of mitochondria in muscle cells, a subject with a muscle tissue disorder, a subject with induced expression of a muscle tissue disorder-inducing gene, and/or a subject with improved (facilitated) apoptosis of muscle cells.

When the preparation for suppressing muscle mass loss of the present invention is used *in vivo,* the subject to be administered is preferably a subject suffering from muscle mass loss. Further, since the preparation for suppressing muscle mass loss of the present invention can suppress the muscle mass loss in age-related muscle loss to be described below, it is preferable that the subject to be administered be a subject suffering from muscle mass loss due to aging. The age-related muscle loss is caused, for example, in middle-aged and elderly people. For this reason, the subjects to be administered are preferably middle-aged or elderly people. For example, the middle-aged and elderly people include people over 40, over 50, over 60, over 70, or over 80.

The use conditions (administration conditions) of the preparation for suppressing muscle mass loss of the present invention are not particularly limited, and for example, the dosage form, the administration timing, the dosage, and the like can be appropriately set according to the type of the subject to be administered, and the like.

Examples of the method for administering the preparation for suppressing muscle mass loss of the present invention include intracerebral administration, intrathecal administration, intramuscular administration, subcutaneous administration, and intravenous administration, and intravenous administration is preferable, for example, because it can safely and stably administer regardless of the skill of the administrator.

The dosage of the preparation for suppressing muscle mass loss of the present invention is the amount of cell that can achieve the effect (therapeutic effect) of suppressing muscle mass loss on the disease of the subject as compared with a subject who has not been administered. Specifically, the dosage can be appropriately determined according to, for example, the age, weight, symptoms, and the like of the subject. Specifically, the dosage may be, for example, 10⁴ to 10⁹ cells/kg weight, 10⁴ to 10⁸ cells/kg weight, or 10⁴ to 10⁷ cells/kg weight, and is preferably 10⁴ to 10⁸ cells/kg weight or 10⁴ to 10⁷ cells/kg weight per administration as the number of umbilical cord-derived cells. The dosage may be, for example, 10⁴ to 10⁹ cells, 10⁶ to 10⁹ cells, 10⁴ to 10⁸ cells, or 10⁴ to 10⁷ cells, and is preferably 10⁴ to 10⁸ cells or 10⁴ to 10⁷ cells per administration as the number of umbilical cord-derived cells. The dosage can also be referred to as, for example, the number of umbilical cord-derived cells in the cell preparation.

The number of administrations of the preparation for suppressing muscle mass loss of the present invention is one or plurality of times. The plurality of times are, for example, 2 times, 3 times, 4 times, 5 times or more. The number of administrations may be determined as appropriate while checking the therapeutic effect of the subject. In the case of the plurality of administrations, the administration interval can be appropriately determined while checking the therapeutic effect of the subject, and may be once daily, once weekly, once biweekly, once monthly, once every three months, or once every six months.

The preparation for suppressing muscle mass loss of the present invention may be used in combination with, for example, other agents and/or methods for use in suppressing muscle mass loss. Examples of the method for use in suppressing muscle mass loss include exercise therapy such as resistance exercise, and reference can be made to the following References 1 to 3 as specific examples. These references are herein incorporated by reference.
Reference 1: Hidetaka WAKABAYASHI, " Exercise Therapy for Sarcopenia", 2013, Japan Medical Journal, No. 4677, pages 32-36
Reference 2: Hidenori Arai et.al., "Special Issue: Clinical Guidelines for Sarcopenia. Guest Editor: Hidenori Arai. This publication has been supported by The Japanese Association on Sarcopenia and Frailty, The Japan Geriatrics Society and National Center for Geriatrics and Gerontology (NCGG) (Japan) Chapter 4 Treatment of sarcopenia", 2018, Geriatrics & Gerontology International, pages 28-44
Reference 3: Masafumi Kuzuya et.al., "Special Issue: Clinical Guide for Frailty. Guest Editors: Shosuke Satake and Hidenori Arai. This publication has been supported by The Japanese Association on Sarcopenia and Frailty, The Japan Geriatrics Society and National Center for Geriatrics and Gerontology (NCGG) (Japan) Chapter 3 Frailty prevention", 2020, Geriatrics & Gerontology International, pages 20-24

The preparation for suppressing muscle mass loss of the present invention can be suitably used for a subject suffering from muscle mass loss, and specifically can be used for a subject suffering from age-related muscle loss. The age-related muscle loss refers to, for example, a symptom or disease of losing muscle mass of skeletal muscle or the like with aging. Examples of the age-related muscle loss include sarcopenia and frailty.

As described above, the preparation for suppressing muscle mass loss of the present invention can suppress muscle mass loss in a subject. Thus, the present invention may include a method for suppressing muscle mass loss in a subject. In this case, the present invention provides a method for suppressing muscle mass loss in a subject, including: using a cell preparation for use in suppressing muscle mass loss of the present invention in a subject. The method for suppressing muscle mass loss of the present invention includes, for example, administering the cell preparation for use in suppressing muscle mass loss of the present invention to the subject. Regarding the administration conditions in the administering, reference can be made to the aforementioned description.

As described above, the preparation for suppressing muscle mass loss of the present invention can suppress muscle mass loss in a subject. Thus, the present invention provides a method for treating a subject suffering from muscle mass loss, including: using the cell preparation for use in suppressing muscle mass loss of the present invention against a subject. A method for treating a subject of the present invention includes, for example, administering the cell preparation for use in suppressing muscle mass loss of the present invention to the subject. Regarding the administration conditions in the administering, reference can be made to the aforementioned description.

As described above, since the preparation for suppressing muscle mass loss of the present invention can suppress the muscle mass loss in a subject, for example, age-related muscle loss can be treated. Therefore, the present invention provides a method for treating a patient with age-related muscle loss, including: administering the cell preparation for use in suppressing muscle mass loss of the present invention to the patient with age-related muscle loss. Regarding the administration conditions in the administering, reference can be made to the aforementioned description.

As used herein, the term "treatment" may be used as the meaning of any of suppression, prevention, restrain, prophylaxis, or delay of the onset of a target disease; stop, suppression, restrain, or delay of the progression of the onset target disease or a symptom thereof; and amelioration or remission of the target disease.

### <Use of Cell Preparation>

In another embodiment, the present invention provides a cell preparation for use in improving a mitochondrial function in muscle tissue or a method for improving a mitochondrial function in muscle tissue. In this case, the present invention provides a cell preparation for use in improving a mitochondrial function in muscle tissue, including: umbilical cord-derived cells. The present invention also provides a method for improving a mitochondrial function in muscle tissue of a subject, including: using the cell preparation for use in improving a mitochondrial function in muscle tissue of the present invention against a subject. Reference can be made to the description as to the cell preparation for use in suppressing the muscle mass loss of the present invention.

In another embodiment, the present invention provides a cell preparation for use in increasing the number of mitochondria in muscle tissue or a method for increasing the number of mitochondria in muscle tissue. In this case, the present invention provides a cell preparation for use in increasing the number of mitochondria in muscle tissue, including: umbilical cord-derived cells. The present invention also provides a method for increasing the number of mitochondria in muscle tissue of a subject, including: using a cell preparation for use in increasing the number of mitochondria in muscle tissue against a subject. Reference can be made to the description as to the cell preparation for use in suppressing the muscle mass loss of the present invention.

In another embodiment, the present invention provides a cell preparation for use in inducing expression of a mitochondrial function-improving gene in muscle tissue or a method for inducing expression of a mitochondrial function-improving gene in muscle tissue. In this case, the present invention provides a cell preparation for use in inducing expression of a mitochondrial function-improving gene in muscle tissue, including: umbilical cord-derived cells. The present invention also provides a method for inducing expression of a mitochondrial function-improving gene in muscle tissue of a subject, including: using a cell preparation for use in inducing expression of a mitochondrial function-improving gene in muscle tissue against a subject. Reference can be made to the description as to the cell preparation for use in suppressing the muscle mass loss of the present invention.

In another embodiment, the present invention provides a cell preparation for use in suppressing apoptosis of muscle cells or a method for suppressing apoptosis of muscle cells. In this case, the present invention provides a cell preparation for use in suppressing apoptosis of muscle cells, including: umbilical cord-derived cells. The present invention also provides a method for suppressing apoptosis of muscle cells of a subject, including: using a cell preparation for use in suppressing apoptosis of muscle cells against a subject. Reference can be made to the description as to the cell preparation for use in suppressing the muscle mass loss of the present invention.

In another embodiment, the present invention provides a cell preparation for use in anti-inflammation in muscle tissue or a method for suppressing inflammation in muscle tissue. In this case, the present invention provides a cell preparation for use in anti-inflammation (inflammation suppression) in muscle tissue, including: umbilical cord-derived cells. The present invention also provides a method for suppressing inflammation in muscle tissue of a subject, including: using a cell preparation for use in anti-inflammation in muscle tissue against a subject. Reference can be made to the description as to the cell preparation for use in suppressing the muscle mass loss of the present invention.

In another embodiment, the present invention provides a cell preparation for use in suppressing expression of an inflammatory cytokine gene and/or a chemokine gene in muscle tissue, or a method for suppressing expression of an inflammatory cytokine gene and/or a chemokine gene in muscle tissue. In this case, the present invention provides a cell preparation for use in suppressing expression of an inflammatory cytokine gene and/or a chemokine gene in muscle tissue, including: umbilical cord-derived cells. The present invention also provides a method for suppressing expression of an inflammatory cytokine gene and/or a chemokine gene in muscle tissue of a subject, including: using a cell preparation for use in suppressing expression of an inflammatory cytokine gene and/or a chemokine gene in muscle tissue against a subject. Reference can be made to the description as to the cell preparation for use in suppressing the muscle mass loss of the present invention.

In another embodiment, the present invention provides a cell preparation for use in promoting proliferation of muscle cells or a method for promoting proliferation of muscle cells. In this case, the present invention provides a cell preparation for use in promoting proliferation of muscle cells, including: umbilical cord-derived cells. The present invention also provides a method for promoting proliferation of muscle cells of a subject, including: using a cell preparation for use in promoting proliferation of muscle cells against a subject. Reference can be made to the description as to the cell preparation for use in suppressing the muscle mass loss of the present invention.

In another embodiment, the present invention provides a cell preparation for use in inducing expression of a muscle cell proliferation-promoting gene or a method for inducing expression of a muscle cell proliferation-promoting gene. In this case, the present invention provides a cell preparation for use in inducing expression of a muscle cell proliferation-promoting gene, including: umbilical cord-derived cells. The present invention also provides a method for inducing expression of a muscle cell proliferation-promoting gene of a subject, including: using a cell preparation for use in inducing expression of a muscle cell proliferation-promoting gene against a subject. Reference can be made to the description as to the cell preparation for use in suppressing the muscle mass loss of the present invention.

In another embodiment, the present invention provides a cell preparation for use in repairing muscle tissue or a method for repairing muscle tissue. In this case, the present invention provides a cell preparation for use in repairing muscle tissue, including: umbilical cord-derived cells. The present invention provides a method for repairing muscle tissue of a subject, including: using a cell preparation for use in repairing muscle tissue against a subject. Reference can be made to the description as to the cell preparation for use in suppressing the muscle mass loss of the present invention.

In another embodiment, the present invention provides a cell preparation for use in inducing expression of a muscle tissue-repairing gene or a method for inducing expression of a muscle tissue-repairing gene. In this case, the present invention provides a cell preparation for use in inducing expression of a muscle tissue-repairing gene, including: umbilical cord-derived cells. The present invention also provides a method for inducing expression of a muscle tissue-repairing gene of a subject, including: using a cell preparation for use in inducing expression of a muscle tissue-repairing gene against a subject. Reference can be made to the description as to the cell preparation for use in suppressing the muscle mass loss of the present invention.

In another embodiment, the present invention provides a cell preparation for use in suppressing muscle tissue disorder or a method for suppressing muscle tissue disorder. In this case, the present invention provides a cell preparation for use in suppressing muscle tissue disorder, including: umbilical cord-derived cells. The present invention also provides a method for suppressing muscle tissue disorder of a subject, including: using a cell preparation for use in suppressing muscle tissue disorder against a subject. Reference can be made to the description as to the cell preparation for use in suppressing the muscle mass loss of the present invention.

In another embodiment, the present invention provides a cell preparation for use in suppressing expression of a muscle tissue disorder-inducing gene or a method for suppressing expression of a muscle tissue disorder-inducing gene. In this case, the present invention provides a cell preparation for use in suppressing expression of a muscle tissue disorder-inducing gene, including: umbilical cord-derived cells. The present invention also provides a method for suppressing expression of a muscle tissue disorder-inducing gene of a subject, including: using a cell preparation for use in suppressing expression of a muscle tissue disorder-inducing gene against a subject. Reference can be made to the description as to the cell preparation for use in suppressing the muscle mass loss of the present invention.

In another embodiment, the present invention provides a cell preparation for use in suppressing interstitial fibrosis in muscle tissue of a subject or a method for suppressing interstitial fibrosis in muscle tissue of a subject. In this case, the present invention provides a cell preparation for use in suppressing interstitial fibrosis in muscle tissue of a subject, including: umbilical cord-derived cells. The present invention also provides a method for suppressing interstitial fibrosis in muscle tissue of a subject, including: using a cell preparation for use in suppressing interstitial fibrosis in muscle tissue of a subject against a subject. Reference can be made to the description as to the cell preparation for use in suppressing the muscle mass loss of the present invention.

### <Use of Cell Preparation>

The present invention provides a cell composition for use in suppressing muscle mass loss, treating age-related muscle loss, improving a mitochondrial function in muscle tissue, increasing the number of mitochondria in muscle tissue, inducing expression of a mitochondrial function-improving gene in muscle tissue, suppressing apoptosis of muscle cells, suppressing inflammation in muscle tissue, suppressing expression of an inflammatory cytokine gene and/or a chemokine gene in muscle tissue, promoting proliferation of muscle cells, inducing expression of a muscle cell proliferation-promoting gene, repairing muscle tissue, inducing expression of a muscle tissue-repairing gene, suppressing muscle tissue disorder, and/or suppressing expression of a muscle tissue disorder-inducing gene, wherein the cell preparation includes umbilical cord-derived cells. The present invention provides the use of umbilical cord-derived cells for producing a cell composition for use in suppressing muscle mass loss, treating age-related muscle loss, improving a mitochondrial function in muscle tissue, increasing the number of mitochondria in muscle tissue, inducing expression of a mitochondrial function-improving gene in muscle tissue, suppressing apoptosis of muscle cells, suppressing inflammation in muscle tissue, suppressing expression of an inflammatory cytokine gene and/or a chemokine gene in muscle tissue, promoting proliferation of muscle cells, inducing expression of a muscle cell proliferation-promoting gene, repairing muscle tissue, inducing expression of a muscle tissue-repairing gene, suppressing muscle tissue disorder, and/or suppressing expression of a muscle tissue disorder-inducing gene. Reference can be made to the description as to the cell preparation for use in suppressing the muscle mass loss of the present invention.

### Examples

Hereinafter, the present invention will be described in detail with reference to examples. It is to be noted, however, that the present invention is not limited to the aspects described in the examples. Unless otherwise indicated, commercially available reagents and kits were used according to the protocol.

### Example 1

It was examined that sarcopenia can be treated by the cell preparation of the present invention.

### (1) Preparation of Umbilical Cord-Derived Cells

Umbilical cord-derived cells were collected by the method described in Cytotherapy,18, 229-241, 2016, which is herein incorporated by reference. Specifically, with the approval of the Ethics Committee of the Institute of Medical Science, University of Tokyo, all tissue elements of the umbilical cord (including amniotic membrane, blood vessels, perivascular tissue, and Wharton jelly) collected with the consent of the donor were shredded into 1 to 2 mm³ fragments and seeded into culture dishes. Then, umbilical cord-derived cells were obtained by a modified explant method of covering with Ceramigo (manufactured by Tsubakimoto Chain Co.) and culturing in α-minimal essential medium (αMEM) supplemented with 10% fetal bovine serum (FBS) and antibiotics. The cells have plastic adherence properties.

### (2) Examination of Surface Antigen

Next, with respect to the obtained umbilical cord-derived cells, whether the surface antigen was expressed was examined. Whether the surface antigen was expressed was examined by FACS analysis using specific antibodies to the respective antigens. As to the surface antigens of umbilical cord-derived cells, CD73, CD105, CD90, CD44 and HLA-class I were positive and HLA-Class II, CD34, CD45, CD19, CD80, CD86, CD40 and CD11b were negative. In addition, HLA-G5 and PD-L2 were positive. HLA-G was weakly positive, CD49d (ITGA4) and CD184 (CXCR4) were negative to weakly positive, and CD29 (ITGB1) was positive.

It was examined by Real-time PCR that the umbilical cord-derived cells expressed a hepatic growth factor (HGF) gene at a high level under normal conditions and that the expression of an indoleamine 2,3-dioxygenase (IDO) gene was induced under inflammatory conditions (IFN-γ 100 ng/ml). In particular, the expression of HGF was found to be higher in umbilical cord-derived cells compared to bone marrow-derived mesenchymal stem cells. In addition, it was examined by ELISA that secretion of PGE2 was induced by co-culturing umbilical cord-derived cells with mixed lymphocyte reaction (MLR).

### (3) Preparation of Cell Preparation

The obtained umbilical cord-derived cells were seeded into Corning^{®} CellBIND^{®} surface 100 mm dishes (manufactured by CORNING, Product Number: #3292), passaged from Passage 1 to Passage 4, and allowed to proliferate. As the culture solution for the passage culture, a medium obtained by adding CiMS^{™}-sAF (manufactured by Nipro Co., Ltd., Product Code: 87-072), which is a human mesenchymal stem cell basal culture solution animal-free additive, to CiMS^{™}-BM (manufactured by Nipro Co., Ltd., Product Code: 87-070), which is a human mesenchymal stem cell basal culture solution, at a proportion according to the package insert was used. The cells were then peeled off using TrypLE^{™} Select Enzyme (1X), no phenol red (ThermoFisher, Product Number: 12563011), and prepared to achieve 1× 10⁶ cell/150 µl to provide the cell preparation of the present invention.

### (4) Model Animal

As an animal to be tested, SAMP10 (purchased from Chubu Kagaku Shizai Co.,Ltd.), which is a Senescence-Accelerated Mouse Prone (SAMP) line mouse showing accelerated aging and short life span, was used. The SAMP10 is a line having trait disorders due to aging such as learning/memory disorder, affective disorder, aging amyloidosis, and the like where cerebral atrophy appear in the early stage. SAMP10 was used as a model animal for sarcopenia because its life span is as short as about 1 year and aging phenomena can be seen from about 24 weeks of age.

### (5) Preparation of Mice

24-week-old SAMP10 mice were randomly picked up and tail vein injection was performed using a BD Roads^{™} 30G syringe with fixed needle for insulin injection (Becton Dickinson, Cat. No. 326638) so as to achieve 1 × 10⁶ cell/mouse, thereby obtaining the mice of Examples. 24-week-old SAMP10 mice administered with the same volume of saline or culture solution were used as controls.

In addition, the mice of Examples and the mice of Control were divided into a group (Ex) to be trained and a group to be not trained. That is, the group (Cell) to which the cells were administered and to be not trained was defined as Example A (n = 8), the group (Cell+Ex) to which the cells were administered and to be trained was defined as Example B (n = 8). A group to which the cells were not administered and to be not trained was defined as a control (n = 7), and a group (Ex) to which the cells were not administered and to be trained was defined as a reference (n = 7). The training was treadmill training three times a week. The treadmill training is training for forcibly running a mouse by using a training apparatus (a treadmill for mouse, manufactured by Melquest Ltd.) provided with a belt conveyor and a device for generating electrical stimulation. In addition, the training contents were changed according to the age of the mice. Specifically, 24- to 26-week-old mice were subjected to a 5-minute warm-up at a velocity of 7 m/min, then to a 35-minute exercise at a velocity of 17 m/min, and to a 5-minute cool-down at a velocity of 7 m/min, with the inclination of the apparatus set at 0°. 27- to 36-week-old mice were subjected to a 5-minute warm-up at a velocity of 10 m/min, then to a 35-minute exercise at a velocity of 18 m/min, and to a 5-minute cool-down at a velocity of 10 m/min, with the inclination of the apparatus set at 5°.

### (6) Results

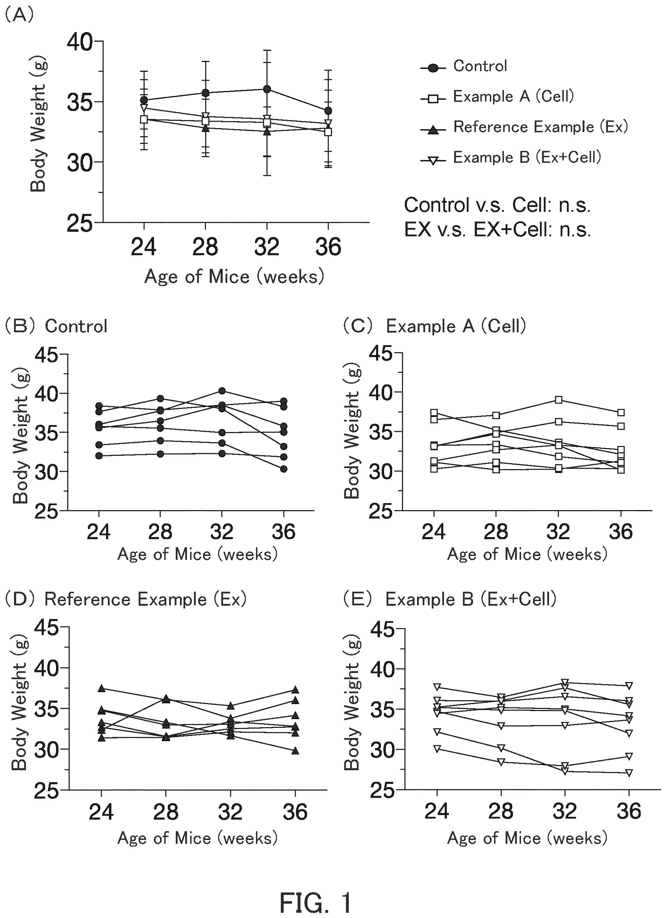

The weights of mice in each group were measured at 24-week-old, 28-week-old, 32-week-old, and 36-week-old. The results are shown in FIG. 1. FIG. 1 shows graphs showing the change in body weight. In each of the graphs of FIG. 1, the vertical axis represents the mean body weight (g) of the mice of each group, and the horizontal axis represents the age in weeks of the mice. In FIG. 1, (A) is a graph comparing the changes in body weight of mice among respective groups, wherein the plot of filled circle indicates a control (Control), the plot of open square indicates Example A (Cell), the plot of filled triangle indicates Reference Example (Ex), and the plot of inverted open triangle indicates Example B (Ex+Cell). In FIG. 1, (B) is a graph showing the body weight of mice of Control (Control), (C) is a graph showing the body weight of mice of Example A (Cell), (D) is a graph showing the body weight of mice of Reference Example (Ex), and (E) is a graph showing the body weight of mice of Example B (Ex+Cell). As shown in FIG. 1, there was no significant difference in body weight of the mice of the respective groups.

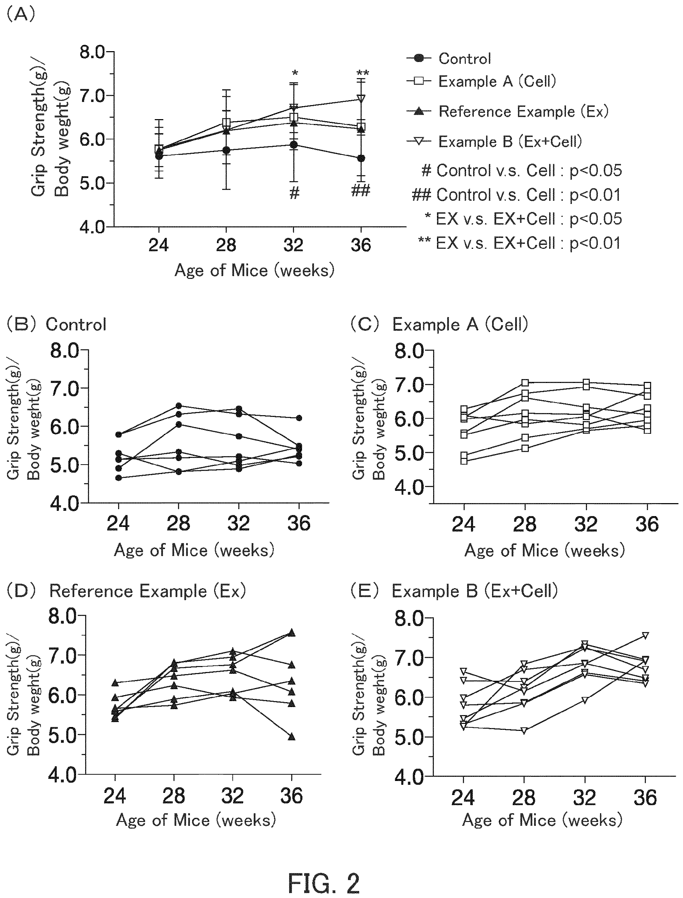

The grip strength of mice in each group was measured at 24weeksold, 28weeksold, 32weeksold, and 26weeksold. The grip strength was measured using a commercially available small animal grip strength measuring device (mouse grip strength meter, manufactured by AMETEK Chatillon) in the following manner. First, a mouse was placed on a net of the device and allowed to hold the net by a limb. From this state, the tail of the mouse was held and pulled horizontally, the force (weight) required for the mouse to let go of the net was measured, and the force immediately before the mouse let go of the net was determined as the grip force. The results are shown in FIG. 2.

FIG. 2 shows graphs showing the change in grip strength per body weight. In each of the graphs of FIG. 2, the vertical axis represents the mean value of grip strength per body weight (grip strength (g)/body weight (g)) in the mice of respective groups, and the horizontal axis represents the age in weeks of the mice. In FIG. 2, (A) is a graph comparing the grip strength (grip strength (g)/body weight (g)) per body weight of mice among respective groups, wherein the plot of filled circle indicates a control, the plot of open square indicates Example A (Cell), the plot of filled triangle indicates Reference Example (Ex), and the plot of inverted open triangle indicates Example B (Ex+Cell). In FIG. 2, (B) is a graph showing the grip strength of mice of Control, (C) is a graph showing the grip strength of mice of Example A (Cell), (D) is a graph showing the grip strength of mice of Reference Example (Ex), and (E) is a graph showing the grip strength of mice of Example B (Ex+Cell).

As shown in FIG. 2, the mice of Example A had significantly improved grip strength at 32 and 36weeks old compared to that of the mice of Control. In addition, the mice of Example B had significantly improved grip strength at 32 and 36weeks old compared to that of the mice of Reference Example (Ex) and the mice of Control. Thus, it was found that the cell preparation of the present invention brings about an effect of improving the grip strength, that is, brings about a therapeutic effect against a decrease in muscle strength in age-related muscle loss.

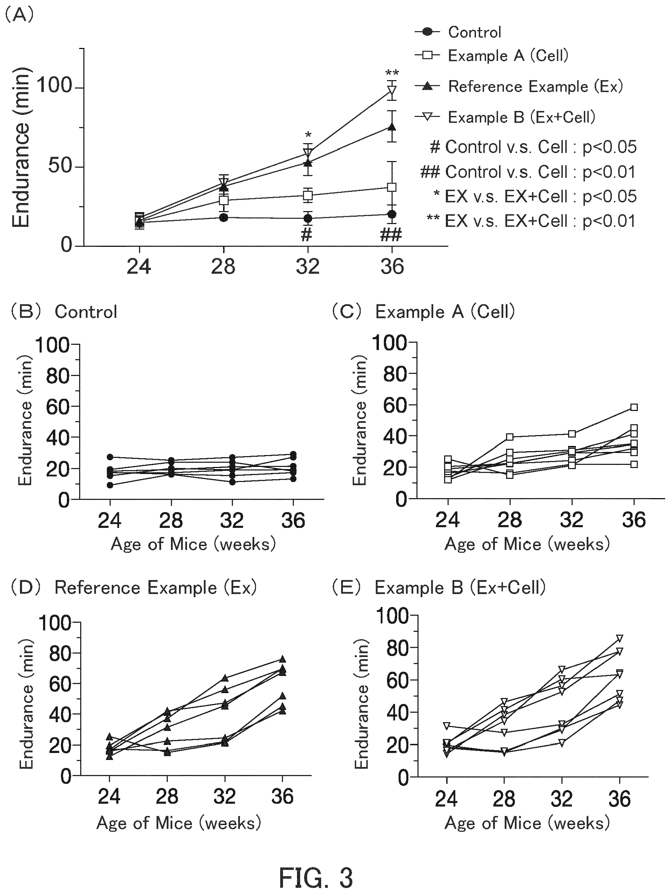

The endurance of mice in each group were measured at 24weeksold, 28weeksold, 32weeksold, and 36weeksold. The endurance was measured by the following endurance measurement method. The results are shown in FIG. 3.

### (Endurance Measurement Method)

| | Speed | Inclination | Time |
|---|---|---|---|
| Start | 6 m/min | 0° | 0 to 5 minutes |
| | 6 m/min | 10° | 5 to 7 minutes |
| | 8 m/min | 10° | 7 to 9 minutes |
| | 10 m/min | 10° | 9 to 11 minutes |
| | 12 m/min | 10° | 11 to 13 minutes |
| | 14 m/min | 10° | 13 to 15 minutes |
| | 16 m/min | 10° | 15 to 17 minutes |
| | 18 m/min | 10° | 17 to 19 minutes |
| | 20 m/min | 10° | 19 to 21 minutes |
| | 21 m/min | 10° | 21 minutes and above |

First, the mice were allowed to run for 5 minutes at a velocity of 6 m/min, with the inclination of the apparatus set at 0°. After 5 minutes, the inclination was changed to 10°, and the mice were allowed to run for 2 minutes at a velocity of 6 m/min, with the inclination of the apparatus set at 10°, and then the velocity was increased by 2 m/min every 2 minutes. The maximum velocity was 21 m/min, and the times until the mice could not run were measured.

FIG. 3 shows graphs showing the endurance. In each of the graphs of FIG. 3, the vertical axis represents the mean value of the endurance (min) in the mice of each group, and the horizontal axis represents the age in weeks of the mice. In FIG. 3, (A) is a graph comparing the endurance (min) of mice among respective groups, wherein the plot of filled circle indicates Control, the plot of open square indicates Example A (Cell), the plot of filled triangle indicates Reference Example (Ex), and the plot of inverted open triangle indicates Example B (Ex+Cell). In FIG. 3, (B) is a graph showing the endurance of mice of Control (Control), (C) is a graph showing the endurance of mice of Example A (Cell), (D) is a graph showing the endurance of mice of Reference Example (Ex), and (E) is a graph showing the endurance of mice of Example B (Ex+Cell).

As shown in FIG. 3, the mice of Example A had significantly improved endurance at 32 weeks old and further improved endurance at 36 weeks old compared to that of the mice of Control. In addition, the mice of Example B had significantly improved endurance at 32 weeks old and further improved endurance at 36 weeks old compared to that of the mice of Reference Example (Ex) and the mice of Control. Thus, it was found that the cell preparation of the present invention brings about an effect of improving endurance, that is, brings about a therapeutic effect against a decrease in muscle strength in age-related muscle loss.

### Example 2

It was examined that the muscle fiber size was improved by administration of the cell preparation of the present invention.

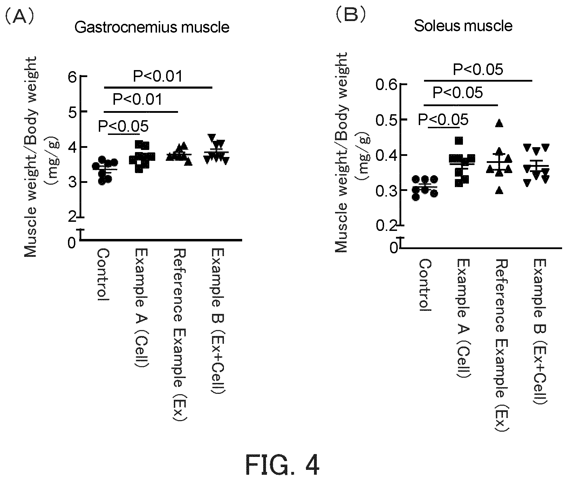

The gastrocnemius and soleus muscles were collected by dissection of the mice used in Example 1, and the weight was measured and the morphology was observed. The results are shown in FIG. 4.

FIG. 4 shows graphs showing the measurement results of the muscle weight. In FIG. 4, (A) is a graph showing the weight of the gastrocnemius muscle of each mouse, and (B) is a graph showing the weight of the soleus muscle of each mouse. In each of the graphs of FIG. 4, the vertical axis represents the relative value of the muscle mass (muscle mass (mg)/body weight (g)) per body weight of the mouse, and the horizontal axis represents the type of the mouse. As shown in FIG. 4, the mice of Example A had significantly increased weights of gastrocnemius and soleus muscles at 12 weeks after cell administration compared to the mice of Control. The mice of Example B had significantly increased gastrocnemius and soleus muscle weights at 12 weeks after cell administration compared to the mice of Control.

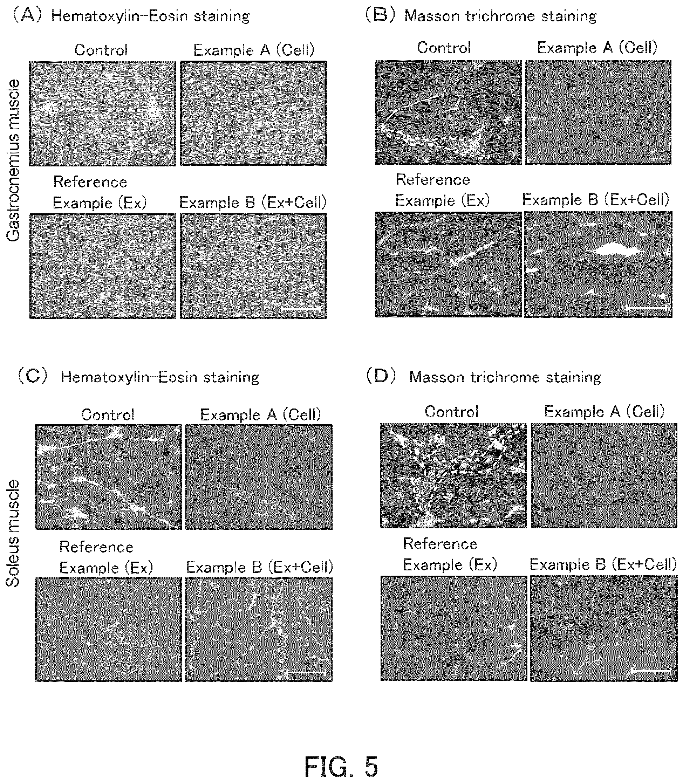

Then, each of the collected muscle tissues was subjected to the Hematoxylin-Eosin (HE) staining to observe morphologically. In addition, each of the collected muscle tissues was subjected to the Masson trichome (MT) staining to examine the severity of interstitial fibrosis of each muscle tissue. The results are shown in FIG. 5.

FIG. 5 shows photographs showing the staining results of the muscle tissue. In FIG. 5, (A) and (B) show the results of the gastrocnemius muscle, wherein (A) is the result of the HE staining, and (B) is the result of the MT staining. In FIG. 5, (C) and (D) show the results of the soleus muscle, wherein (C) is the result of the HE staining and (D) is the result of the MT staining. In (B) and (D) of FIG. 5, the locations stained blue by the MT staining, i.e., the interstitial fibrosis area, are surrounded by broken lines. As shown in FIG. 5, the mice of Example A showed increased muscle fiber size of the gastrocnemius and soleus muscles at 12 weeks after the administration of the cells as compared with the mice of Control. The mice of Example B showed increased muscle fiber size of the gastrocnemius and soleus muscles at 12 weeks after cell administration compared to the mice of Control. In addition, the mice of Examples A and B showed decreased interstitial fibrosis in both the gastrocnemius muscle and the soleus muscle compared to the mice of Control. It is known that a decrease in muscle cross-sectional area (muscle fiber size), interstitial fibrosis, and the like are caused in patients with age-related muscle loss such as sarcopenia and frailty. Since the cell preparation of the present invention can improve muscle cross-sectional area and suppress interstitial fibrosis, it is presumed that the cell preparation of the present invention has a therapeutic effect on age-related muscle loss.

From the above, it was found that the cell preparation of the present invention increases the muscle mass by improving the muscle cross-sectional area and suppressing the interstitial fibrosis, and thereby achieving a therapeutic effect on the age-related muscle loss.

### Example 3

It was examined that the administration of the cell preparation of the present invention showed an effect of improving the mitochondrial function.

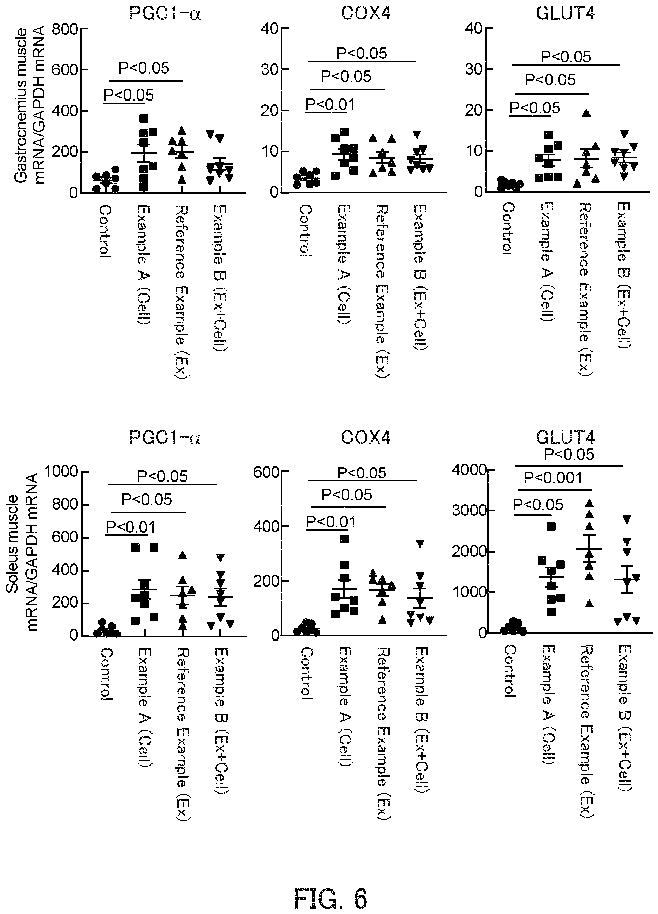

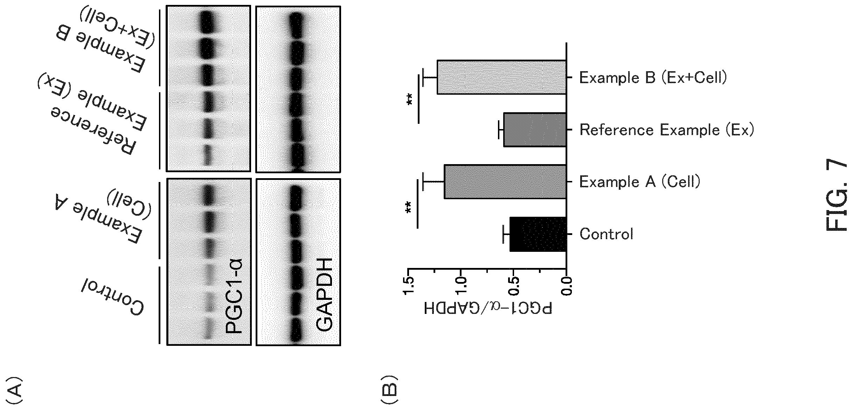

With resepetct to the gastrocnemius and soleus muscles of each of the mice collected in Example 2, the expression level of the mRNA encoding PGC1-α, COX4, and GLUT4 in each muscle tissue was examined by a RT-PCR method using a qPCR kit (PowersSYBRR Green CR Master Mix, ThermoFisher Co., Cat. No.: #437659). The expression level of PGC1-α in the gastrocnemius muscle was examined by Western blotting. The expression level of PGC1-α was calculated from the obtained gel stained image. As endogenous controls of the RT-PCR and the Western blotting, glyceraldehyde phosphate dehydrogenase (GAPDH) was used (hereinafter, the same). These results are shown in FIGs. 6 and 7.
- Primer set for PGC1-α
   Forward primer: 5'-CCGAGAATTCATGGAGCAAT-3 ' (SEQ ID NO: 1)
   Reverse primer: 5'-TTTCTGTGGGTTTGGTGTGA-3' (SEQ ID NO: 2)
- Primer set for COX4
   Forward primer: 5'-AGCTGAGCCAAGCAGAGAAG-3 ' (SEQ ID NO: 3)
   Reverse primer: 5'-AATCACCAGAGCCGTGAATC-3' (SEQ ID NO: 4)
- Primer set for GLUT4
   Forward primer: 5'-GACGGACACTCCATCTGTTG-3 ' (SEQ ID NO: 5)
   Reverse primer: 5'-GCCACGATGGAGACATAGC-3' (SEQ ID NO: 6)
- Primer set for GAPDH
   Forward primer: 5'-ATGTGTCCGTCGTGGATCTGA-3 ' (SEQ ID NO: 7)
   Reverse primer: 5'-ATGCCTGCTTCACCACCTTCT-3' (SEQ ID NO: 8)

FIG. 6 shows graphs showing the expression levels of PGC1-α, COX4, and GLUT4 in the gastrocnemius and soleus muscles of each of the mice. In FIG. 6, three graphs in the upper row are graphs showing the results of the gastrocnemius muscle, and three graphs in the lower row are graphs showing the results of the soleus muscle. In the graphs in the upper and lower rows in FIG. 6, the results of PGC1-α, COX4, and GLUT4 are shown from the left. In each of the graphs of FIG. 6, the vertical axis indicates the relative value of the expression level of the mRNA of PGC1-α, COX4 or GLUT4 to the endogenous control (GAPDH), and the horizontal axis indicates the type of the mouse.

FIG. 7 shows photographs showing the results of the Western blotting. In FIG. 7, (A) shows gel images showing the result of PGC1-α in the gastrocnemius muscle, and (B) is a graph showing the expression level of PGC1-α in the gastrocnemius muscle. In the gel images of (A) of FIG. 7, the upper row indicates the expression level of PGC1-α, and the lower row indicates the expression level of the endogenous control (GAPDH). In the gel images of (A) of FIG. 7, the results of Control, Example A (Cell), Reference Example (Ex), and Example B (Cell+Ex) are shown for each of the three lanes from the left.

As shown in FIG. 6, the mice of Example A showed increased expression levels of the mRNA of PGC1-α, COX4, and GLUT4 in the gastrocnemius and soleus muscles at 12 weeks after administration of the cells compared to the mice of Control. The mice of Example B showed increased expression levels of the mRNA of PGC1-α, COX4, and GLUT4 in the gastrocnemius and soleus muscles at 12 weeks after administration of the cells compared to the mice of Control.

As shown in (A) and (B) of FIG. 7, the mice of Example A showed increased expression level of PGC1-α-protein in gastrocnemius and soleus muscles at 12 weeks after administration of cells compared to the mice of Control. The mice of Example B showed increased expression level of PGC1-α protein in the gastrocnemius muscle at 12 weeks after administration of the cells as compared with the mice of Control and Reference Examples. From these results, it was presumed that the cell preparation of the present invention suppressed the muscle mass loss through the unique function of improving the mitochondrial function.

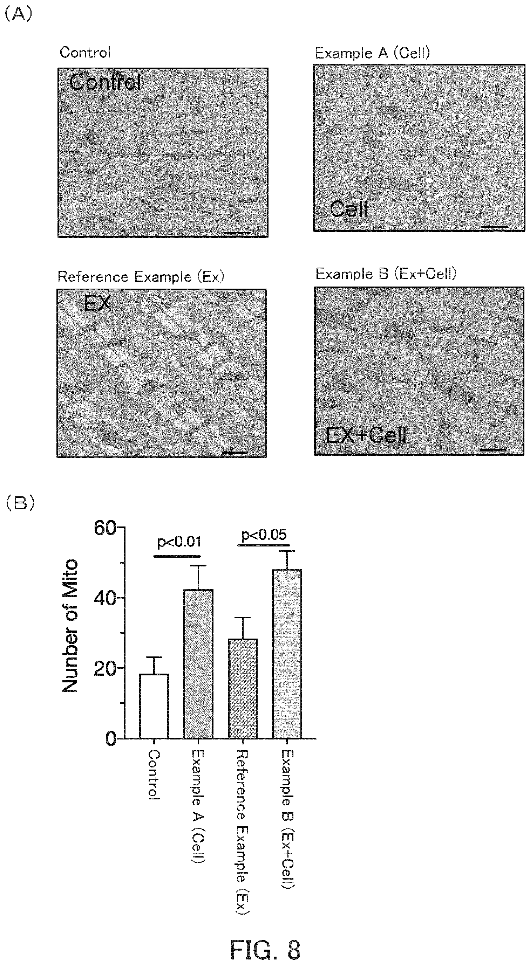

Next, the morphology of the mitochondria in the gastrocnemius muscle was observed using an electron microscope (JEM-1400Plus, manufactured by JEOL Ltd.). The number of mitochondria per unit area was then counted. The results are shown in FIG. 8.

FIG. 8 shows photographs and a graph showing the measurement results of the mitochondria. In FIG. 8, (A) shows the results of electron microscopy images of the gastrocnemius muscle, and (B) shows the results of the number of mitochondria per unit area. As shown in (A) and (B) of FIG. 8, the mice of Examples A and B showed increased number of mitochondria in the gastrocnemius muscle at 12 weeks after the administration of the cells compared to the mice of Control and Reference Examples.

It has been suggested that a decrease in a mitochondrial function is associated with age-related muscle loss such as sarcopenia and frailty. Therefore, it is presumed that the cell preparation of the present invention brings about a therapeutic effect on age-related muscle loss through the mitochondrial function-improving effect.

### Example 4

It was examined that apoptosis of muscle cells was suppressed by administration of the cell preparation of the present invention.

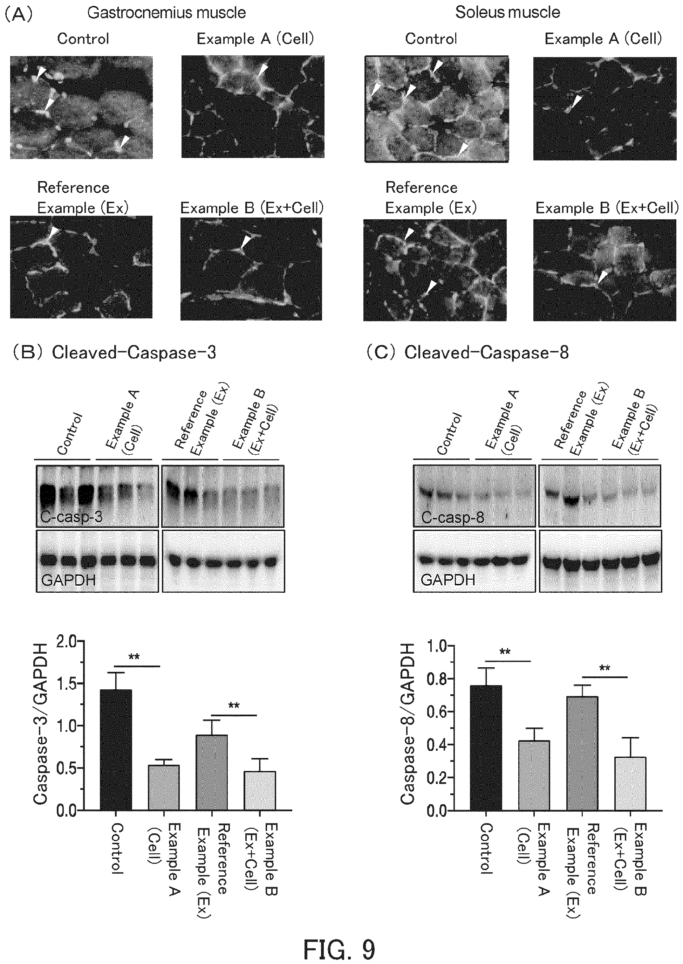

With respect to the gastrocnemius and soleus muscles of the mice collected in Example 2, the amounts of fragmented DNA showing apoptosis of cells were examined in the respective muscle tissues by a T-mediated digoxygenin(biotin)-dUTP nick end labeling (TUNEL) method. In addition, the expression levels of Cleaved-caspase-3 and Cleaved-caspase-8, which are markers of endogenous apoptosis, were examined by Western blotting. The results are shown in FIG. 9.

FIG. 9 shows photographs and graphs showing the results of the apoptosis analysis. In FIG. 9, (A) shows photographs showing the result of the TUNEL staining, wherein four photographs on the left show the results of the gastrocnemius muscle, and four photographs on the right show the results of the soleus muscle. In four photographs on the left and right of (A) of FIG. 9, the upper left shows the result of Control, the upper right shows the result of Example A (Cell), the lower left shows the result of Reference Example (Ex), and the lower right shows the result of Example B (Cell+Ex). In (A) of FIG. 9, the locations of the fragmented DNA stained by the TUNEL staining are indicated by inverted open triangles. In FIG. 9, (B) shows gel images and a graph showing the expression level of Cleaved-caspase-3 in the gastrocnemius muscle. In FIG. 9, (C) shows gel images and a graph showing the expression level of Cleaved-caspase-8 in the gastrocnemius muscle. In each of the graphs of (B) and (C) of FIG. 9, the vertical axis indicates the relative value of the expression level of Cleaved-caspase-3 or Cleaved-caspase-8 to the endogenous control (GAPDH), and the horizontal axis indicates the type of the mouse. As shown in FIG. 9, mice of Examples A and B showed decreased fragmented DNA indicating apoptosis of cells in muscle tissues at 12 weeks after administration of cells compared to the mice of Control. In addition, the mice of Examples A and B showed decreased expression levels of Cleaved-caspase-3 and Cleaved-caspase-8, which are markers of endogenous apoptosis in muscle tissues at 12 weeks after administration of cells, compared to the mice of Control and Reference Examples.

It is known that the apoptosis of muscle cells is facilitated in patients with sarcopenia. It was found that the cell preparation of the present invention can suppress, for example, apoptosis of muscle cells, and thus can suppress muscle mass loss, thereby achieving a therapeutic effect of sarcopenia. In addition, since muscle mass is reduced by apoptosis of muscle cells also in age-related muscle loss such as frailty, the cell preparation of the present invention can be said to have a therapeutic effect even on other age-related muscle loss.

### Example 5

It was examined that administration of the cell preparation of the present invention showed an anti-inflammatory effect.

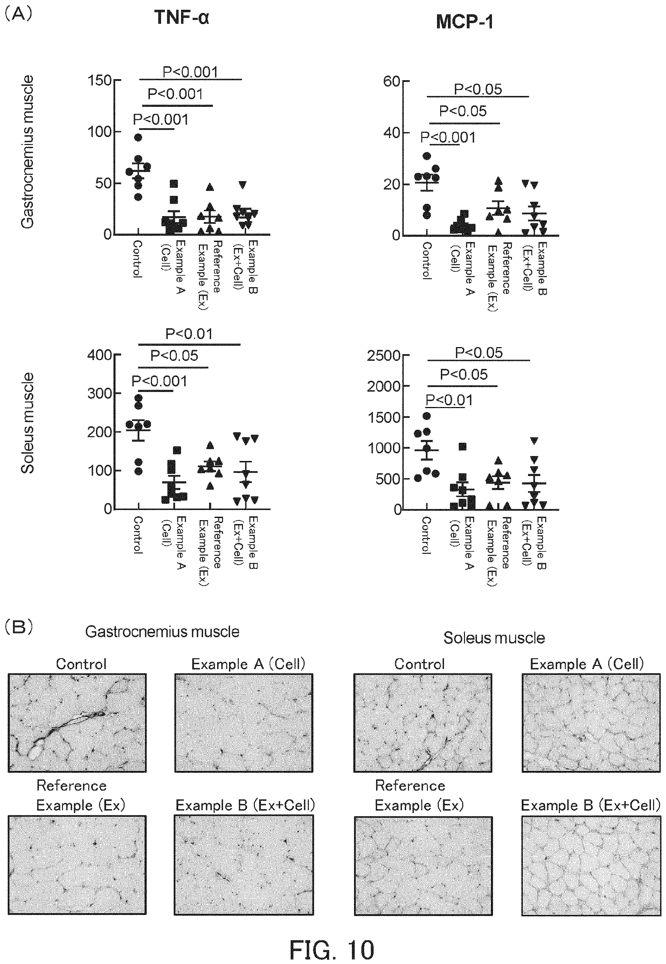

With respect to the gastrocnemius and soleus muscles of the mice collected in Example 2, the expression levels of the mRNA of Tumor necrosis factor-α (TNF-α), which is an inflammatory cytokine, and Monocyte chemotactic protein-1 (MCP-1), which is a chemokine, in each muscle tissue were examined by a RT-PCR method using a qPCR kit (PowersSYBRR Green CR Master Mix, Cat.No.: #437659, manufactured by ThermoFisher). In addition, the collected muscle tissues were subjected to the CD68 staining to detect macrophages. The results are shown in FIG. 10.
- Primer set for TNF-α
   Forward primer: 5'-GACTTTCTCCTGGTATGAGATAG-3 ' (SEQ ID NO: 9)
   Reverse primer: 5'-AGGCTGCCCCGACTACGT-3' (SEQ ID NO: 10)
- Primer set for MCP-1
   Forward primer: 5'-GCCCCACTCACCTGCTGCTACT-3 ' (SEQ ID NO: 11)
   Reverse primer: 5'-CCTGCTGCTGGTGATCCTCTTGT-3' (SEQ ID NO: 12)

FIG. 10 shows graphs showing the expression level of the inflammatory cytokine and photographs showing the muscle tissue. In FIG. 10, (A) shows graphs showing the expression levels of TNF-α or MCP-1 in the gastrocnemius and soleus muscles of mice. In (A) of FIG. 10, two graphs in the upper row show the results in the gastrocnemius muscle, and two graphs in the lower row show the results in the soleus muscle. In the upper and lower graphs of (A) of FIG. 10, TNF-α and MCP-1 are shown from the left. In each of the graphs of (A) of FIG. 10, the vertical axis represents the expression level of the mRNA of TNF-α or MCP-1 relative to the endogenous control (GAPDH), and the horizontal axis represents the type of the mouse. In FIG. 10, (B) shows photographs showing the results of the CD68 staining, wherein four photographs on the left show the results of the gastrocnemius muscle, and four photographs on the right show the results of the soleus muscle. In four photographs on the left and right of (B) of FIG. 10, the upper left shows the result of Control, the upper right shows the result of Example A (Cell), the lower left shows the result of Reference Example (Ex), and the lower right shows the result of Example B (Cell+Ex).

As shown in FIG. 10, the mice of Examples A and B showed decreased expression levels of the mRNA of TNF-α or MCP-1 in the gastrocnemius and soleus muscles at 12 weeks after administration of the cells compared to the mice of Control. From the results of the CD68 staining, it was found that the amount of macrophage in the gastrocnemius and soleus muscles in the mice of Examples A and B was lower than that of the mice of Control.

Since the expression levels of TNF-α and MCP-1 in muscle tissue and the amount of macrophage in muscle tissue are decreased in mice of Examples A and B, it was found that the cell preparation of the present invention can suppress the inflammation of the muscle tissue. As aging progresses, fine inflammation occurs in muscle tissue, which is said to decrease muscle mass. Thus, it was found that the cell preparation of the present invention can suppress the muscle mass loss by suppressing the inflammation of the muscle tissue, thereby achieving a therapeutic effect on sarcopenia.

### Example 6

It was examined that the proliferation of muscle cells was improved by administration of the cell preparation of the present invention.

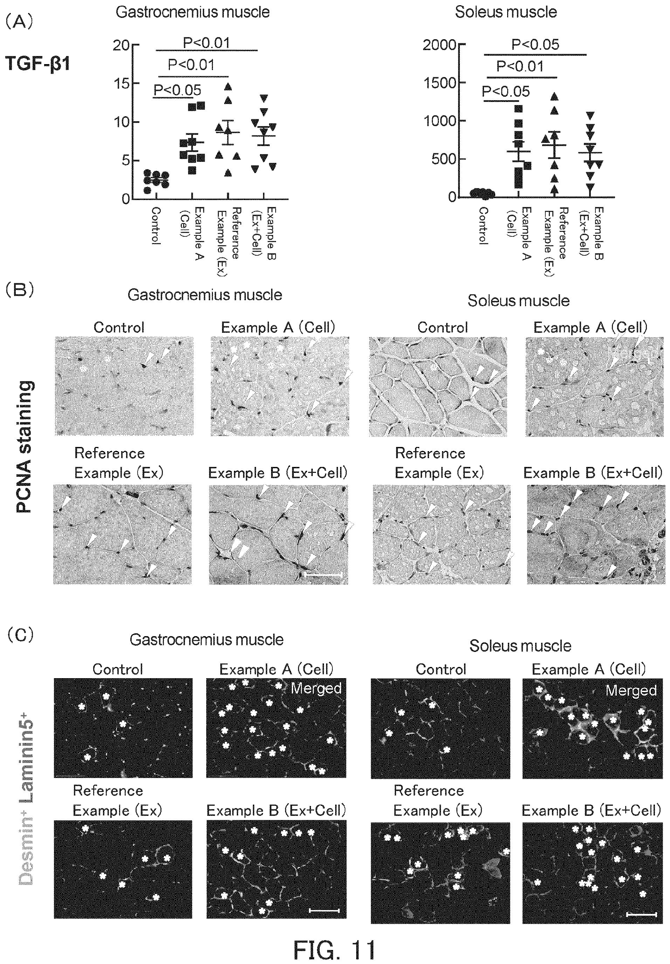

With respect to the gastrocnemius and soleus muscles of the mice collected in Example 2, the expression level of the mRNA of TGF-β1 in muscle tissue was examined by a RT-PCR method using a qPCR kit (PowersSYBRR Green CR Master Mix, Cat.No.: #437659, manufactured by ThermoFisher). In addition, the collected muscle tissues were subjected to the proliferating cell nuclear antigen (PCNA) staining to detect proliferating cell nuclear antigens in muscle tissues, and then subjected to the Desmin+/Laminin5+ fluorescence double fluorescent staining to examine the expression of desmin and laminin 5 in muscle tissues. The results are shown in FIG. 11.
- Primer set for TGF-β1
   Forward primer: 5'-TGGAGCAACATGTGGAACTC-3 ' (SEQ ID NO: 13)
   Reverse primer: 5'-GTCAGCAGCCGGTTACCA-3' (SEQ ID NO: 14)

FIG. 11 shows graphs showing the expression level of TGF-β1 and photographs showing the muscle tissue. In FIG. 11, (A) shows graphs showing the expression levels of TGF-β1 in the gastrocnemius and soleus muscles of the mice, and (B) shows photographs showing the results of the PCNA staining. In (A) of FIG. 11, the graph on the left shows the results in the gastrocnemius muscle, and the graph on the right shows the results in the soleus muscle. In each of the graphs of (A) of FIG. 11, the vertical axis represents the expression level of the mRNA of TGF-β1 relative to the endogenous control (GAPDH), and the horizontal axis represents the type of the mouse. In (B) of FIG. 11, four photographs on the left show the results of the gastrocnemius muscle, and four photographs on the right show the results of the soleus muscle. In four photographs on the left and right of (B) of FIG. 11, the upper left shows the result of Control, the upper right shows the result of Example A (Cell), the lower left shows the result of Reference Example (Ex), and the lower right shows the result of Example B (Cell+Ex). In (B) of FIG. 11, the locations of the proliferating cell nuclear antigen stained by the PCNA staining are indicated by inverted open triangles. In FIG. 11, (C) shows photographs showing the results of the fluorescent double fluorescent staining of Desmin/Laminin5, wherein four photographs on the left show the results of the gastrocnemius muscle, and four photographs on the right show the results of the soleus muscle. In four photographs on the left and right of (C) of FIG. 11, the upper left shows the result of Control, the upper right shows the result of Example A (Cell), the lower left shows the result of Reference Example (Ex), and the lower right shows the result of Example B (Cell+Ex). In (C) of FIG. 11, the locations of Desmin⁺/Laminin5⁺ (double positive) stained by the fluorescence double fluorescent staining of Desmin/Laminin5 are indicated by white asterisks (*).

As shown in FIG. 11, the mice of Examples A and B showed increased expression levels of the mRNA of TGF-β1 in the gastrocnemius and soleus muscles at 12 weeks after administration of cells compared to the mice of Control. From the results of the PCNA staining, it was found that the number of proliferating cell nuclear antigens in the gastrocnemius and soleus muscles were increased, that is, the proliferation of skeletal muscle cells was facilitated at 12 weeks after the administration of the cells compared to the mice of Control. Furthermore, from the results of the Desmin+/Laminin5+ fluorescence double fluorescence staining, it was found that the mice of Examples A and B showed increased expression levels of Desmin and Laminin5 in the gastrocnemius and soleus muscles at 12 weeks after the administration of cells, i.e., showed induction of repair of skeletal muscle, compared to the mice of Control and Reference Examples.

From these results, it was found that the cell preparation of the present invention induces the proliferation of skeletal muscle cells, thereby promoting the repair of skeletal muscle. In addition, since TGF-β1 induces the proliferation of skeletal muscle cells, it was presumed that the cell preparation of the present invention induces the above-described pathway through the induction of the expression of TGF-β1. It is known that the number of skeletal muscle cells is reduced in patients with age-related muscle loss such as sarcopenia and frailty. Thus, it was found that the cell preparation of the present invention vegetates the muscle mass loss by inducing cell proliferation of skeletal muscle, and thereby achieving a therapeutic effect on age-related muscle loss.

### Example 7

It was examined that the administration of the cell preparation of the present invention facilitates the repair of skeletal muscle.

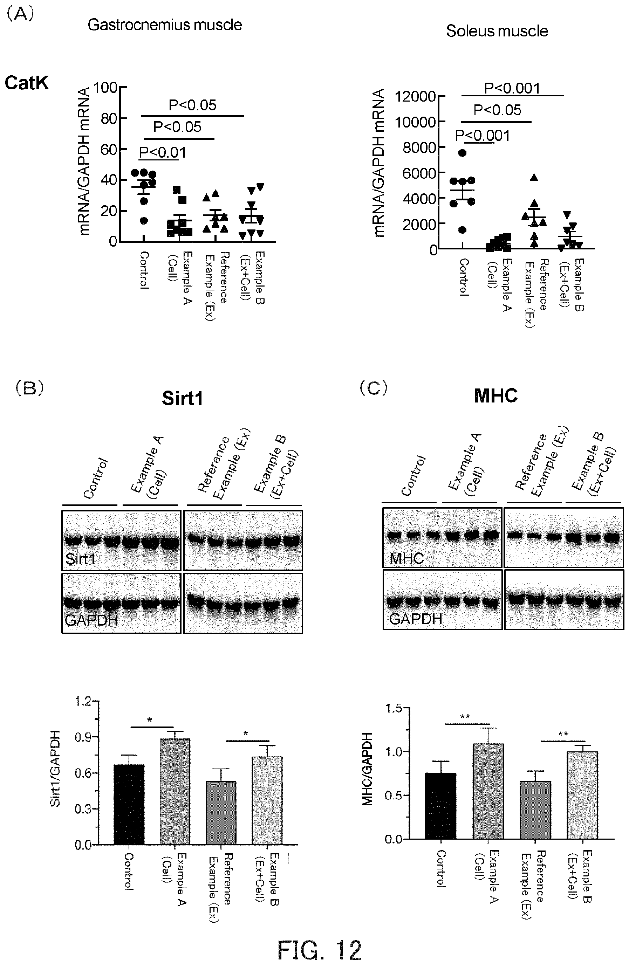

With respect to the gastrocnemius and soleus muscles of the mice collected in Example 2, the expression levels of the mRNA encoding cathepsin K (CatK) and GAPDH were examined by a RT-PCR method using a qPCR kit (PowersSYBRR Green CR Master Mix, Cat.No.: #437659, manufactured by ThermoFisher). The expression levels of Sirt1 and myosin heavy chain (MHC) and the expression level of GAPDH were examind by Western blotting. The results are shown in FIG. 12.
- Primer set for CatK
   Forward primer: 5'-AGCAGGCTGGAGGACTAAGGT-3 ' (SEQ ID NO: 15)
   Reverse primer: 5'-TTTGTGCATCTCAGTGGAAGACT-3' (SEQ ID NO: 16)

FIG. 12 shows graphs and photographs showing the expression levels of CatK, Sirt1, and MHC. In FIG. 12, (A) shows graphs showing the expression level of CatK in the gastrocnemius and soleus muscles of mice, (B) shows gel images and a graph showing the expression level of Sirt1 in the gastrocnemius muscle, (C) shows gel images and a graph showing the expression level of MHC in the gastrocnemius muscle. In (A) of FIG. 12, the graph on the left shows the results of the gastrocnemius muscle, and the graph on the right shows the results of the soleus muscle. In each of the graphs in (A) of FIG. 12, the vertical axis indicates the relative value of the expression level of CatK to the endogenous control (GAPDH), and the horizontal axis indicates the type of the mouse. In the gel images of (B) and (C) of FIG. 12, the upper row indicates the expression level of Sirt1 or MHC, and the lower row indicates the expression level of endogenous control (GAPDH). Further, in the gel images, the results of Control, Example A (Cell), Reference Example (Ex), and Example B (Cell+Ex) are shown for each of the three lanes from the left. In the graphs of (B) and (C) of FIG. 12, the vertical axis represents the relative value of the expression level of Sirt1 or MHC to the endogenous control (GAPDH), and the horizontal axis represents the type of the mouse.

As shown in (A) of FIG. 12, the mice of Examples A and B showed significantly decreased expression levels of CatK in muscle tissues at 12 weeks after the administration of the cells compared to the mice of Control. In addition, the mice of Examples A and B showed significantly increased expression levels of Sirt1 and MHC at 12 weeks after administration of the cells compared to the mice of Control and Reference Examples.

CatK is known to be highly expressed after a muscle disorder and to potentiate a muscle disorder by provoking inflammation and delay regeneration. In addition, it is known that a sirtuin gene such as Sirt1 is a gene related to an anti-aging effect. MHC is known to be a gene associated with the repair and enhancement of muscle mass and to have the function of maintaining the muscle mass fibers. Therefore, it was presumed that since the cell preparation of the present invention can suppress the expression level of CatK in skeletal muscle and increase the expression level of Sirt1 and MHC, for example, the cell preparation of the present invention can facilitate and maintain the repair skeletal muscle. Thus, it was found that the cell preparation of the present invention suppresses the expression of a gene that potentiates a muscle disorder and, on the other hand, suppresses the muscle mass loss by inducing the expression of a gene that regenerates muscle, thereby achieving a therapeutic effect on sarcopenia.

### Example 8

It was examined that the exosomes of the cell preparation of the present invention can suppress apoptosis of muscle cells.

### (1) Purification of Exosomes from Cell Preparation Culture Supernatant

In order to examine that the exosomes of the cell preparation of the present invention suppress apoptosis, first, the exosomes were purified from the cell preparation culture supernatant. Specifically, the cell preparations prepared in the same manner as in Examples 1(1) to (3) were seeded into Corning^{®} CellBIND^{®} surface 100 mm dishes (manufactured by CORNING, Inc., Product Number: #3292) so as to achieve 1× 10⁵ cell/1 ml, and cultured for 48 hours. As the culture solution for the culture, a medium obtained by adding CiMS^{™}-sAF (manufactured by Nipro Co., Ltd., Product Code: 87-072), which is a human mesenchymal stem cell basal culture solution animal-free additive, to CiMS^{™}-BM (manufactured by Nipro Co., Ltd., Product Code: 87-070) at a proportion according to the package insert was used. After the culturing, the first centrifugation was performed at 300×g for 10 minutes to remove the cells, thereby obtaining the first supernatant. The supernatant was subjected to the second centrifugation at 2000×g for 10 minutes to remove the cell fragments and the like, thereby obtaining a second supernatant. The second supernatant was then filtered using a 0.22 µm filter to obtain a sample. The sample was placed in a UC tube for ultracentrifugation, placed on a rotor (SW32Ti-12U, manufactured by Beckman Coulter, Inc.) and subjected to the third centrifugation using an ultracentrifuge (Optima L-100, manufactured by Beckman Coulter, Inc.). The third centrifugation was performed at 35000 rpm for 70 minutes. After the third centrifugation, the supernatant was removed to obtain a precipitate. The PBS, which has been filtered using a 1 ml filter, was added to the precipitate, and the resultant was subjected to vortexing. After the vortexing, the resultant was diluted in a tube to achieve 35 ml using a PBS, and then subjected to the fourth centrifugation under the same conditions as in the third centrifugation. After the fourth centrifugation, the supernatant was removed, a small amount of PBS was added, and vortexing was performed to obtain an exosome solution. The exosome solution was then stored in a 1.5 ml low adsorption tube.

### (2) Electron Microscope Examination of Exosomes

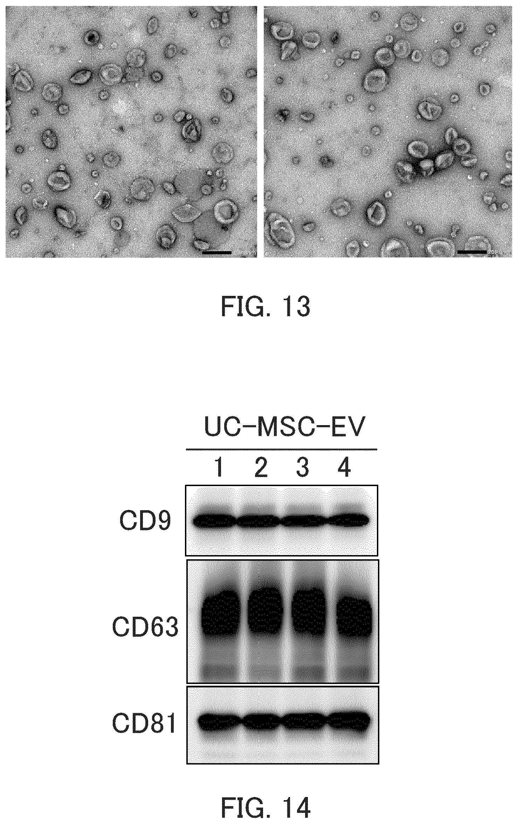

Next, in order to examine whether exosomes are contained in the exosome solution, the exosome solution was observed by an electron microscope. Specifically, 10 µl of the exosome solution was placed on a grid and subjected to natural drying for 10 minutes. After the natural drying, uranyl acetate was added and a negative staining was performed. After the negative staining, observations were performed using a transmission electron microscope (JEM-1400PLUS, manufactured by JEOL Ltd.). The results are shown in FIG. 13.

FIG. 13 shows photographs of the exosome by the electron microscope. In FIG. 13, the scale bar indicates 200 nm. As shown in FIG. 13, a large number of round vesicles having diameters of approximately from about 50 nm to about 200 nm were observed. From the above, it was suggested that exosomes can be purified from the culture supernatant of the cell preparation of the present invention.

### (3) Examination of Exosome Markers

In order to examine whether the round vesicles observed by the electron microscope were exosomes, Western blotting was used to examine whether the vesicles expressed exosome markers. Specifically, 50 µl of 2× sample buffer was added to 50 µl of the exosome solution obtained in Example 8(1), and the mixture was heated at 95°C for 5 minutes to prepare a sample. After the preparation, the samples were applied to 10-20% SuperSep (TM) Ace (manufactured by FUJIFILM Wako Pure Chemical Corporation) acrylamide gel wells and electrophoresed. After the electrophoresis, the Western blotting was performed. Detection of exosomes in the Western blotting was performed by reacting primary antibodies overnight, followed by staining with secondary antibodies at room temperature (about 25°C) for 1 hour. As the primary antibody, an anti-CD9 monoclonal antibody (clone: 1K, 1000-fold dilution, Cat.No:041-27763, manufactured by FUJIFILM Wako Pure Chemical Corporation), an anti-CD63 monoclonal antibody (clone: 3-13, 1000-fold dilution, Cat.No: 012-27063, manufactured by FUJIFILM Wako Pure Chemical Corporation), and an anti-CD81 monoclonal antibody (clone:17B1, 1000-fold dilution, Cat.No: 011-27773, manufactured by FUJIFILM Wako Pure Chemical Corporation), which are markers of exosomes, were used. As the secondary antibody, an AntiMouse IgG, HRP-Linked F(ab') ₂ Fragment Sheep (5000-fold dilution, Cat.No: NA9310V, manufactured by Cytiva) was used. In the same manner as described above, the exosome markers were detected in three exosome solutions that were independently and additionally prepared. The results are shown in FIG. 14.

FIG. 14 shows photographs showing the results of detection of exosome markers by the Western blotting. In FIG. 14, the names of the exosome markers are indicated on the left of the photographs and the name of the exosome solution sample is indicated above the photographs. In FIG. 14, lanes 1 to 4 each show the result of an independently prepared exosome solution. As shown in FIG. 14, exosome markers were detected in all samples of the exosome solution, and it was verified that umbilical cord-derived mesenchymal stem cells secreted exosomes with good reproducibility. From the above, it was found that exosomes can be purified from the culture supernatant of the cell preparation of the present invention.

### (4) Evaluation of Particle Size Distribution of Exosomes

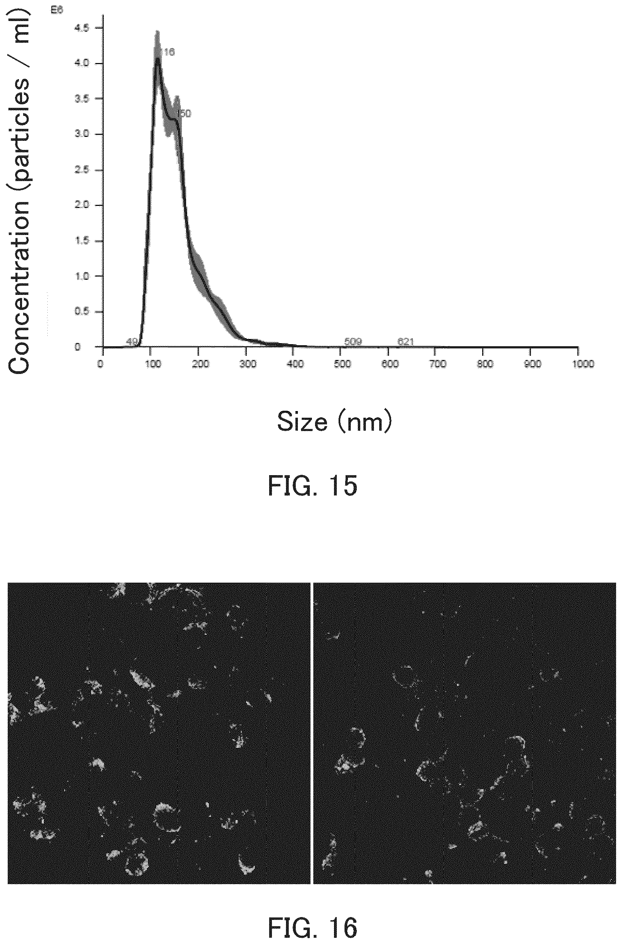

The particle size distribution of exosomes obtained from the culture supernatant of the cell preparation of the present invention was evaluated. Specifically, the exosome solution obtained in Example 8 (1) was diluted 50-fold using PBS. After the dilution, the exosome particle size was measured using a particle size analyzer (Nano particle tracking analysis (NanoSight), manufactured by Malvern Panalytical Ltd.) with default parameters. The results are shown in FIG. 15.

FIG. 15 is a graph showing the distribution of the particle size of the exosome marker. In FIG. 15, the vertical axis represents the concentration of exosomes (vesicle number/ml), and the horizontal axis represents the size of exosomes (nm). As shown in FIG. 15, the particle sizes of the exosomes of the umbilical cord-derived mesenchymal cells were distributed from 40 to 400 nm, with the largest number of 116 nm size exosomes, the smallest 40 nm size exosome, and the largest 621 nm size exosome. Many exosomes of the size from about 100 nm to about 300 nm were seen. From the above, it was found that the exosomes obtained from the culture supernatant of the cell preparation of the present invention were exosomes having a size from about 100 nm to about 300 nm.

### (5) Evaluation of Uptake of Exosomes into Cultured Muscle Cells

Evaluation was made as to whether exosomes obtained from the culture supernatant of the cell preparation of the present invention were taken up by cultured muscle cells. Specifically, the exosome solution obtained in Example 8 (1) (10 µg as the amount of protein) was labeled using ExoSparkler Exosome Membrane Labeling Kit-Green (Cat.No:EX01, manufactured by Dojindo Laboratories). After the labeling, the resultant was added to C2C12 cells (murine striated muscle cells) that have been seeded into a dish at 1.25 × 10⁴ cells. After the addition, incubation was performed for 24 hours. After the incubation, the cells were fixed using a 4% paraformaldehyde-containing phosphate buffer (Cat.No: 161-20141, manufactured by FUJIFILM Wako Pure Chemical Corporation) at room temperature for 20 minutes. After the fixing, washing was performed using PBS at room temperature for 5 minutes, and the washing was performed three times in total. Thereafter, the cells were observed using a fluorescent microscope (Super-resolution/confocal Microscopy (LSM880-ELYRAPS. 1, manufactured by Carl Zeiss Corporation)). The results are shown in FIG. 16.

FIG. 16 shows photographs showing the C2C12 cells that have taken up the exosomes. As shown in FIG. 16, C2C12 cells were found to take up labeled exosomes. From the above, it was found that exosomes obtained from the culture supernatant of the cell preparation of the present invention are taken up by muscle cells.

### (6) Evaluation of Suppression of Apoptosis by Exosomes

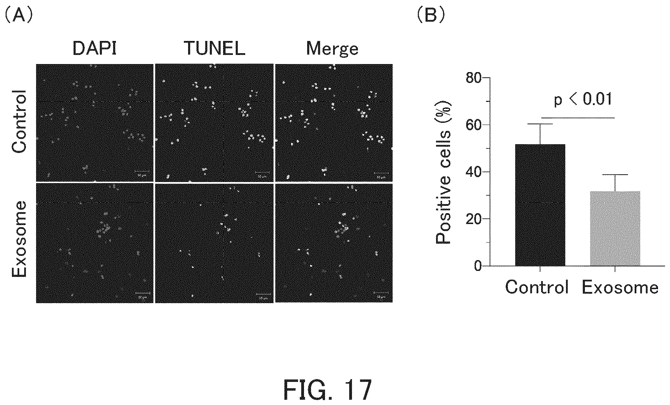

It was examined whether exosomes obtained from the culture supernatant of the cell preparation of the present invention suppress apoptosis of cultured muscle cells that have taken up the exosomes. Specifically, C2C12 cells (murine striated muscle cells) were seeded into a 12-well culture plate containing a coverslip so as to achieve 2×10⁴ cells, and then 300 µmol/l H₂O₂ was added. After the addition, incubation was performed for 12 hours. After the incubation, culture was performed in the DMEM medium supplemented with the exosome solution (1 µg as protein amount) obtained in Example 8 (1). Negative Controls were cultured using a DMEM medium supplemented with an equal volume of PBS instead of the exosome solution. The culture was carried out for 12 hours. After the culturing, the cells were subjected to the TUNEL staining, and observed using a fluorescent microscope. AProLong^{™} Galss Antifade Mountan with NucBlue^{™} (Cat.No: P36985, manufactured by ThermoFisher) was used to stain the nuclei. The proportion of TUNEL positive cells in nucleus positive cells was calculated. The results are shown in FIG. 17. As the test for significant differences, t tests (and nonparametric tests) were used.

FIG. 17 shows photographs and a graph showing the apoptosis of the C2C12 cells that have taken up the exosomes. In FIG. 17, (A) show photographs showing the TUNEL staining with or without the addition of exosomes, and (B) is a graph showing the proportion of TUNEL positive cells with or without the addition of exosomes. In (A) of FIG. 17, whether exosomes were added or not is described on the left of the photographs, and the types of the marker are described above the photographs. In (B) of FIG. 17, the vertical axis represents the proportion (%) of TUNEL positive cells, and the vertical axis represents whether the exosome was added or not. As shown in (A) of FIG. 17, less TUNEL positive cells were observed in C2C12 cells (Exosome) to which exosomes were added as compared with a Negative Control (Control) to which no exosomes were added. As shown in (B) of FIG. 17, in C2C12 cells to which exosomes were added, the proportion of TUNEL positive cells was significantly reduced as compared with the negative control to which no exosomes were added. From the above, it was found that the exosomes obtained from the culture supernatant of the cell preparation of the present invention can suppress apoptosis caused by H₂O₂ in the muscle cells that have taken up the exosomes. It should be noted that the inventors of the present invention have shown for the first time that umbilical cord-derived cells secrete exosomes.

While the invention has been particularly shown and described with reference to embodiments and examples thereof, the invention is not limited to these embodiments and examples. It will be understood by those of ordinary skill in the art that various changes in form and details may be made therein without departing from the spirit and scope of the invention as defined by the claims.

This application claims priority from Japanese Patent Application No. 2020-190551 filed on November 16, 2020. The entire subject matter of the Japanese Patent Applications is incorporated herein by reference.

### SUPPLEMENTARY NOTE

The whole or part of the embodiments and examples disclosed above can be described as, but not limited to, the following supplementary notes.

### (Supplementary Note 1)

A cell preparation for use in suppressing muscle mass loss, comprising:
umbilical cord-derived cells.

### (Supplementary Note 2)

The cell preparation according to Supplementary Note 1, wherein
the umbilical cord-derived cell is an umbilical cord-derived mesenchymal cell.

### (Supplementary Note 3)

The cell preparation according to Supplementary Note 1 or 2, wherein
the umbilical cord-derived cell is
(i) positive for CD105, CD73, CD90, CD44, HLA-class I, HLA-G5, and PD-L2, and
(ii) negative for CD45, CD34, CD11b, CD19, and HLA-Class II.

### (Supplementary Note 4)

The cell preparation according to any one of Supplementary Notes 1 to 3, wherein
the umbilical cord-derived cell
(iii) induces expression of a gene or a protein or both of at least one protein selected from the group consisting of IDO, PGE2, and PD-L1, under an inflammatory condition.

### (Supplementary Note 5)

The cell preparation according to any one of Supplementary Notes 1 to 4, wherein
the umbilical cord-derived cell is a cell prepared from umbilical cord tissue, comprising at least one material selected from the group consisting of amnion, blood vessels, perivascular tissue, and Wharton jelly.

### (Supplementary Note 6)

The cell preparation according to any one of Supplementary Notes 1 to 5, comprising:
1×10⁶ to 1×10⁹ umbilical cord-derived cells.

### (Supplementary Note 7)

The cell preparation according to any one of Supplementary Notes 1 to 6, comprising:
at least one cell selected from the group consisting of an extract of the umbilical cord-derived cell and a secretion of the umbilical cord-derived cell.

### (Supplementary Note 8)

The cell preparation according to any one of Supplementary Notes 1 to 7, wherein the cell preparation exhibits an effect of improving a mitochondrial function in muscle tissue.

### (Supplementary Note 9)

The cell preparation according to any one of Supplementary Notes 1 to 8, wherein the cell preparation exhibits an effect of increasing the number of mitochondria in muscle tissue.

### (Supplementary Note 10)

The cell preparation according to any one of Supplementary Notes 1 to 9, wherein the cell preparation exhibits an effect of inducing expression of a mitochondrial function-improving gene in muscle tissue.

### (Supplementary Note 11)

The cell preparation according to Supplementary Note 10, wherein
the mitochondrial function-improving gene is at least one gene selected from the group consisting of a PGC1-α gene, a COX4 gene, and a GLUT4 gene.

### (Supplementary Note 12)

The cell preparation according to any one of Supplementary Notes 1 to 11, wherein the cell preparation exhibits an effect of suppressing apoptosis of muscle cells.

### (Supplementary Note 13)

The cell preparation according to any one of Supplementary Notes 1 to 12, wherein the cell preparation exhibits an anti-inflammatory effect in muscle tissue.

### (Supplementary Note 14)

The cell preparation according to any one of Supplementary Notes 1 to 13, wherein the cell preparation exhibits an effect of suppressing expression of at least one gene selected from the group consisting of an inflammatory cytokine gene and a chemokine gene, in muscle tissue.

### (Supplementary Note 15)

The cell preparation according to Supplementary Note 14, wherein
the inflammatory cytokine gene is a TNF-α gene.

### (Supplementary Note 16)

The cell preparation according to Supplementary Note 14, wherein
the chemokine gene is a MCP-1 gene.

### (Supplementary Note 17)

The cell preparation according to any one of Supplementary Notes 1 to 16, wherein the cell preparation exhibits an effect of promoting proliferation of muscle cells.

### (Supplementary Note 18)

The cell preparation according to any one of Supplementary Notes 1 to 17, wherein the cell preparation exhibits an effect of inducing expression of a muscle cell proliferation-promoting gene.

### (Supplementary Note 19)

The cell preparation according to Supplementary Note 18, wherein
the muscle cell proliferation-promoting gene is a TGF-β1 gene.

### (Supplementary Note 20)

The cell preparation according to any one of Supplementary Notes 1 to 19, wherein the cell preparation exhibits a muscle tissue-repairing effect.

### (Supplementary Note 21)

The cell preparation according to any one of Supplementary Notes 1 to 20, wherein the cell preparation exhibits an effect of inducing expression of a muscle tissue-repairing gene.

### (Supplementary Note 22)

The cell preparation according to Supplementary Note 21, wherein
the muscle tissue-repairing gene is at least one gene selected from the group consisting of a SIRT1 gene and a MHC gene.

### (Supplementary Note 23)

The cell preparation according to any one of Supplementary Notes 1 to 22, wherein the cell preparation exhibits an effect of suppressing muscle tissue disorder.

### (Supplementary Note 24)

The cell preparation according to any one of Supplementary Notes 1 to 23, wherein the cell preparation exhibits an effect of suppressing expression of a muscle tissue disorder-inducing gene.

### (Supplementary Note 25)

The cell preparation according to Supplementary Note 24, wherein
the muscle tissue disorder-inducing gene is a cathepsin K gene.

### (Supplementary Note 26)

The cell preparation according to any one of Supplementary Notes 1 to 25, for use in combination with exercise therapy.

### (Supplementary Note 27)

The cell preparation according to any one of Supplementary Notes 1 to 26, for use in a subject having inflammation in muscle tissue.

### (Supplementary Note 28)

The cell preparation according to any one of Supplementary Notes 1 to 27, for intravenous administration.

### (Supplementary Note 29)

The cell preparation according to any one of Supplementary Notes 1 to 28, wherein
the muscle mass loss is due to aging.

### (Supplementary Note 30)

The cell preparation according to any one of Supplementary Notes 1 to 29, for administration to middle-aged or elderly people.

### (Supplementary Note 31)

A cell preparation for use in treatment of age-related muscle loss, comprising:
the cell preparation for use in suppressing muscle mass loss according to any one of Supplementary Notes 1 to 30.

### (Supplementary Note 32)

The cell preparation according to Supplementary Note 31, wherein
the age-related muscle loss is sarcopenia or frailty.

### (Supplementary Note 33)

A cell preparation for use in improving a mitochondrial function in muscle tissue, comprising:
umbilical cord-derived cells.

### (Supplementary Note 34)

A cell preparation for use in increasing the number of mitochondria in muscle tissue, comprising:
umbilical cord-derived cells.

### (Supplementary Note 35)

A cell preparation for use in inducing expression of a mitochondrial function-improving gene in muscle tissue, comprising:
umbilical cord-derived cells.

### (Supplementary Note 36)

The cell preparation according to Supplementary Note 35, wherein
the mitochondrial function-improving gene is at least one gene selected from the group consisting of a PGC1-α gene, a COX4 gene, and a GLUT4 gene.

### (Supplementary Note 37)

A cell preparation for use in suppressing apoptosis of muscle cells, comprising:
umbilical cord-derived cells.

### (Supplementary Note 38)

A cell preparation for use in anti-inflammation (inflammation suppression) in muscle tissue, comprising:
umbilical cord-derived cells.

### (Supplementary Note 39)

A cell preparation for use in suppressing expression of an inflammatory cytokine gene and/or a chemokine gene in muscle tissue, comprising:
umbilical cord-derived cells.

### (Supplementary Note 40)

The cell preparation according to Supplementary Note 39, wherein
the inflammatory cytokine gene is a TNF-α gene.

### (Supplementary Note 41)

The cell preparation according to Supplementary Note 39, wherein
the chemokine gene is a MCP-1 gene.

### (Supplementary Note 42)

A cell preparation for use in promoting proliferation of muscle cells, comprising:
umbilical cord-derived cells.

### (Supplementary Note 43)

A cell preparation for use in inducing expression of a muscle cell proliferation-promoting gene, comprising:
umbilical cord-derived cells.

### (Supplementary Note 44)

The cell preparation according to Supplementary Note 43, wherein
the muscle cell proliferation-promoting gene is a TGF-β1 gene.

### (Supplementary Note 45)

A cell preparation for use in repairing muscle tissue, comprising:
umbilical cord-derived cells.

### (Supplementary Note 46)

A cell preparation for use in inducing expression of a muscle tissue-repairing gene, comprising:
umbilical cord-derived cells.

### (Supplementary Note 47)

The cell preparation according to Supplementary Note 46, wherein
the muscle tissue-repairing gene is at least one gene selected from the group consisting of a SIRT1 gene and a MHC gene.

### (Supplementary Note 48)

A cell preparation for use in suppressing muscle tissue disorder, comprising:
umbilical cord-derived cells.

### (Supplementary Note 49)

A cell preparation for use in suppressing expression of a muscle tissue disorder-inducing gene, comprising:
umbilical cord-derived cells.

### (Supplementary Note 50)

The cell preparation according to Supplementary Note 49, wherein
the muscle tissue disorder-inducing gene is a cathepsin K gene.

### (Supplementary Note 51)

A cell preparation for use in suppressing interstitial fibrosis in muscle tissue of a subject, comprising:
umbilical cord-derived cells.

### (Supplementary Note 52)

A method for suppressing muscle mass loss in a subject, comprising:
using the cell preparation for use in suppressing muscle mass loss according to any one of Supplementary Notes 1 to 30 against a subject.

### (Supplementary Note 53)

The method according to Supplementary Note 52, comprising:
administering the cell preparation for use in suppressing muscle mass loss according to any one of Supplementary Notes 1 to 30 to the subject.

### (Supplementary Note 54)

A method for treating a subject suffering from muscle mass loss comprising:
using the cell preparation for suppressing muscle mass loss according to any one of Supplementary Notes 1 to 30 against the subject.

### (Supplementary Note 55)

The method according to Supplementary Note 54, comprising:
administering the cell preparation for use in suppressing muscle mass loss according to any one of Supplementary Notes 1 to 30 to the subject.

### (Supplementary Note 56)

A method for improving a mitochondrial function in muscle tissue of a subject, comprising:
using the cell preparation for use in improving a mitochondrial function in muscle tissue according to Supplementary Note 33.

### (Supplementary Note 57)

The method according to Supplementary Note 56, comprising:
administering the cell preparation according to Supplementary Note 33 to a subject.

### (Supplementary Note 58)

A method for increasing the number of mitochondria in muscle tissue of a subject, comprising:
using the cell preparation for use in increasing the number of mitochondria in muscle tissue according to Supplementary Note 34 against the subject.

### (Supplementary Note 59)

The method according to Supplementary Note 58, comprising:
administering the cell preparation according to Supplementary Note 34 to the subject.

### (Supplementary Note 60)

A method for inducing expression of a mitochondrial function-improving gene in muscle tissue of a subject, comprising:
using the cell preparation for use in inducing expression of a mitochondrial function-improving gene in muscle tissue according to Supplementary Note 35 or 36 against the subject.

### (Supplementary Note 61)

The method according to Supplementary Note 60, comprising:
administering the cell preparation according to Supplementary Note 35 or 36 to the subj ect.

### (Supplementary Note 62)

A method for suppressing apoptosis of muscle cells of a subject, comprising:
using the cell preparation for use in suppressing apoptosis of muscle cells according to Supplementary Note 37 against the subject.

### (Supplementary Note 63)

The method according to Supplementary Note 62, comprising:
administering the cell preparation according to Supplementary Note 37 to the subject.

### (Supplementary Note 64)

A method for suppressing inflammation in muscle tissue of a subject, comprising:
using the cell preparation for use in anti-inflammation (inflammation suppression) in muscle tissue according to Supplementary Note 38 against the subject.

### (Supplementary Note 65)

The method according to Supplementary Note 64, comprising:
administering the cell preparation according to Supplementary Note 38 to the subject.

### (Supplementary Note 66)

A method for suppressing expression of an inflammatory cytokine gene and/or a chemokine gene in muscle tissue of a subject, comprising:
using the cell preparation for use in suppressing expression of an inflammatory cytokine gene and/or a chemokine gene in muscle tissue according to Supplementary Note 39 against the subj ect.

### (Supplementary Note 67)

The method according to Supplementary Note 66, comprising:
administering the cell preparation according to Supplementary Note 39 to the subject.

### (Supplementary Note 68)

A method for promoting proliferation of muscle cells of a subject, comprising:
using the cell preparation for use in promoting proliferation of muscle cells according to Supplementary Note 42 against the subject.

### (Supplementary Note 69)

The method according to Supplementary Note 68, comprising:
administering the cell preparation according to Supplementary Note 42 to the subject.

### (Supplementary Note 70)

A method for inducing expression of a muscle cell proliferation-promoting gene of a subject, comprising:
using the cell preparation for use in inducing expression of a muscle cell proliferation-promoting gene according to Supplementary Note 43 or 44 against the subject.

### (Supplementary Note 71)

The method according to Supplementary Note 70, comprising:
administering the cell preparation according to Supplementary Note 43 or 44 to a subj ect.

### (Supplementary Note 72)

A method for repairing muscle tissue of a subject, comprising:
using the cell preparation for use in repairing muscle tissue according to Supplementary Note 45 against the subject.

### (Supplementary Note 73)

The method according to Supplementary Note 72, comprising:
administering the cell preparation according to Supplementary Note 45 to the subject.

### (Supplementary Note 74)

A method for inducing expression of a muscle tissue-repairing gene of a subject, comprising:
using the cell preparation for use in inducing expression of a muscle tissue-repairing gene according to Supplementary Note 46 or 47 against the subject.

### (Supplementary Note 75)

The method according to Supplementary Note 74, comprising:
administering the cell preparation according to Supplementary Note 46 or 47 to the subj ect.

### (Supplementary Note 76)

A method for suppressing muscle tissue disorder of a subject, comprising:
using the cell preparation for use in suppressing muscle tissue disorder according to Supplementary Note 48 against the subject.

### (Supplementary Note 77)

The method according to Supplementary Note 76, comprising
administering the cell preparation according to Supplementary Note 48 to the subject.

### (Supplementary Note 78)

A method for suppressing expression of a muscle tissue disorder-inducing gene of a subject, comprising:
using the cell preparation for use in suppressing expression of a muscle tissue disorder-inducing gene according to Supplementary Note 49 or 50 against the subject.

### (Supplementary Note 79)

The method according to Supplementary Note 78, comprising
administering the cell preparation according to Supplementary Note 49 or 50 to the subj ect.

### (Supplementary Note 80)

A method for treating a patient with age-related muscle loss, comprising:
administering the cell preparation according to any one of Supplementary Notes 1 to 30 to the patient with age-related muscle loss.

### (Supplementary Note 81)

A method for suppressing interstitial fibrosis in muscle tissue of a subject, comprising:
using the cell preparation for use in suppressing interstitial fibrosis in muscle tissue of a subject according to Supplementary Note 51 against the subject.

### (Supplementary Note 82)

The method according to Supplementary Note 81, comprising:
administering the cell preparation according to Supplementary Note 51 to the subject.

### (Supplementary Note 83)

A cell composition for use in suppressing muscle mass loss, comprising
umbilical cord-derived cells.

### (Supplementary Note 84)

A cell preparation for use in treating age-related muscle loss, comprising
umbilical cord-derived cells.

### (Supplementary Note 85)

A cell preparation for use in improving a mitochondrial function in muscle tissue, comprising:
umbilical cord-derived cells.

### (Supplementary Note 86)

A cell preparation for use in increasing the number of mitochondria in muscle tissue, comprising:
umbilical cord-derived cells.

### (Supplementary Note 87)

A cell preparation for use in inducing expression of a mitochondrial function-improving gene in muscle tissue, comprising:
umbilical cord-derived cells.

### (Supplementary Note 88)

A cell preparation for use in suppressing apoptosis of muscle cells, comprising:
umbilical cord-derived cells.

### (Supplementary Note 89)

A cell preparation for use in suppressing inflammation in muscle tissue, comprising:
umbilical cord-derived cells.

### (Supplementary Note 90)

A cell preparation for use in suppressing expression of an inflammatory cytokine gene and/or a chemokine gene in muscle tissue, comprising:
umbilical cord-derived cells.

### (Supplementary Note 91)

A cell preparation for use in promoting proliferation of muscle cells, comprising:
umbilical cord-derived cells.

### (Supplementary Note 92)

A cell preparation for use in inducing expression of a muscle cell proliferation-promoting gene, comprising:
umbilical cord-derived cells.

### (Supplementary Note 93)

A cell preparation for use in repairing muscle tissue, comprising
umbilical cord-derived cells.

### (Supplementary Note 94)

A cell preparation for use in inducing expression of a muscle tissue-repairing gene, comprising:
umbilical cord-derived cells.

### (Supplementary Note 95)

A cell preparation for use in suppressing muscle tissue disorder, comprising:
umbilical cord-derived cells.

### (Supplementary Note 96)

A cell preparation for use in suppressing expression of a muscle tissue disorder-inducing gene, comprising:
umbilical cord-derived cells.

### (Supplementary Note 97)

A cell preparation for use in suppressing interstitial fibrosis in muscle tissue, comprising:
umbilical cord-derived cells.

### (Supplementary Note 98)

A pharmaceutical composition, comprising:
exosomes derived from umbilical cord-derived cells; and
a pharmaceutically acceptable carrier.

### (Supplementary Note 99)

The pharmaceutical composition according to Supplementary Note 98, wherein
a mean diameter of the exosome is in a range from 1 to 500 nm.

### (Supplementary Note 100)

The pharmaceutical composition according to Supplementary Note 98 or 99, wherein
the exosome includes a lipid membrane and a lumen,
the lipid membrane is derived from the umbilical cord-derived cell, and
the lumen comprises a cytoplasm of the umbilical cord-derived cell.

### (Supplementary Note 101)

The pharmaceutical composition according to any one of Supplementary Notes 98 to 100, for use in suppressing muscle mass loss.

### (Supplementary Note 102)

The pharmaceutical composition according to any one of Supplementary Notes 98 to 101, for use in improving a mitochondrial function in muscle tissue.

### (Supplementary Note 103)

The pharmaceutical composition according to any one of supplementary notes 98 to 102, for use in increasing the number of mitochondria in muscle tissue

### (Supplementary Note 104)

The pharmaceutical composition according to any one of Supplementary Notes 98 to 103, for use in inducing expression of a mitochondrial function-improving gene in muscle tissue.

### (Supplementary Note 105)

The pharmaceutical composition according to any one of Supplementary Notes 98 to 104 for use in suppressing apoptosis of muscle cells.

### (Supplementary Note 106)

The pharmaceutical composition according to any one of Supplementary Notes 98 to 105, for use in anti-inflammation (inflammation suppression) in muscle tissue.

### (Supplementary Note 107)

The pharmaceutical composition according to any one of Supplementary Notes 98 to 106, for use in suppressing expression of at least one material selected from the group consisting of an inflammatory cytokine gene and a chemokine gene, in muscle tissue.

### (Supplementary Note 108)

The pharmaceutical composition according to any one of Supplementary Notes 98 to 107, for use in promoting proliferation of muscle cells.

### (Supplementary Note 109)

The pharmaceutical composition according to any one of Supplementary Notes 98 to 108, for use in inducing expression of a muscle cell proliferation-promoting gene.

### (Supplementary Note 110)

The pharmaceutical composition according to any one of Supplementary Notes 98 to 109, for use in repairing muscle tissue.

### (Supplementary Note 111)

The pharmaceutical composition according to any one of Supplementary Notes 98 to 110, for use in inducing expression of a muscle tissue-repairing gene.

### (Supplementary Note 112)

The pharmaceutical composition according to any one of Supplementary Notes 98 to 111, for use in suppressing muscle tissue disorder.

### (Supplementary Note 113)

The pharmaceutical composition according to any one of Supplementary Notes 98 to 112, for use in age-related muscle loss.

### Industrial Applicability

As described above, according to the present invention, the muscle mass loss can be suppressed. Thus, according to the present invention, a disease caused by muscle mass loss can be treated. Therefore, the present invention is extremely useful, for example, in the pharmaceutical field.

## Claims

1. A cell preparation for use in suppressing muscle mass loss, comprising:
umbilical cord-derived cells.

2. The cell preparation according to claim 1, wherein
the umbilical cord-derived cell is an umbilical cord-derived mesenchymal cell.

3. The cell preparation according to claim 1 or 2, wherein
the umbilical cord-derived cell is
(i) positive for CD105, CD73, CD90, CD44, HLA-class I, HLA-G5, and PD-L2, and
(ii) negative for CD45, CD34, CD11b, CD19, and HLA-Class II.

4. The cell preparation according to any one of claims 1 to 3, wherein
the umbilical cord-derived cell
(iii) induces expression of a gene or a protein or both of at least one protein selected from the group consisting of IDO, PGE2, or PD-L1 under an inflammatory condition.

5. The cell preparation according to any one of claims 1 to 4, wherein
the umbilical cord-derived cell is a cell prepared from umbilical cord tissue, comprising at least one material selected from the group consisting amnion, blood vessels, perivascular tissue, and Wharton jelly.

6. The cell preparation according to any one of claims 1 to 5, comprising:
1×10⁶ to 1×10⁹ umbilical cord-derived cells.

7. The cell preparation according to any one of claims 1 to 6, comprising:
at least one cell selected from the group consisting of an extract of the umbilical cord-derived cell and a secretion of the umbilical cord-derived cell.

8. The cell preparation according to any one of claims 1 to 7, wherein the cell preparation exhibits an effect of improving a mitochondrial function in muscle tissue.

9. The cell preparation according to any one of claims 1 to 8, wherein the cell preparation exhibits an effect of increasing the number of mitochondria in muscle tissue.

10. The cell preparation according to any one of claims 1 to 9, wherein the cell preparation exhibits an effect of inducing expression of a mitochondrial function-improving gene in muscle tissue.

11. The cell preparation according to claim 10, wherein
the mitochondrial function-improving gene is at least one gene selected from the group consisting a PGC1-α gene, a COX4 gene, and a GLUT4 gene.

12. The cell preparation according to any one of claims 1 to 11, wherein the cell preparation exhibits an effect of suppressing apoptosis of muscle cells.

13. The cell preparation according to any one of claims 1 to 12, wherein the cell preparation exhibits an anti-inflammatory effect in muscle tissue.

14. The cell preparation according to any one of claims 1 to 13, wherein the cell preparation exhibits an effect of suppressing expression of at least one gene selected from the group consisting an inflammatory cytokine gene and a chemokine gene, in muscle tissue.

15. The cell preparation according to claim 14, wherein
the inflammatory cytokine gene is a TNF-α gene.

16. The cell preparation according to claim 14, wherein
the chemokine gene is a MCP-1 gene.

17. The cell preparation according to any one of claims 1 to 16, wherein the cell preparation exhibits an effect of promoting proliferation of muscle cells.

18. The cell preparation according to any one of claims 1 to 17, wherein the cell preparation exhibits an effect of inducing expression of a muscle cell proliferation-promoting gene.

19. The cell preparation according to claim 18, wherein
the muscle cell proliferation-promoting gene is a TGF-β1 gene.

20. The cell preparation according to any one of claims 1 to 19, wherein the cell preparation exhibits a muscle tissue-repairing effect.

21. The cell preparation according to any one of claims 1 to 20, wherein the cell preparation exhibits an effect of inducing expression of a muscle tissue-repairing gene.

22. The cell preparation according to claim 21, wherein
the muscle tissue-repairing gene is at least one gene selected from the group consisting a SIRT1 gene and a MHC gene.

23. The cell preparation according to any one of claims 1 to 22, wherein the cell preparation exhibits an effect of suppressing muscle tissue disorder.

24. The cell preparation according to any one of claims 1 to 23, wherein the cell preparation exhibits an effect of suppressing expression of a muscle tissue disorder-inducing gene.

25. The cell preparation according to claim 24, wherein
the muscle tissue disorder-inducing gene is a cathepsin K gene.

26. The cell preparation according to any one of claims 1 to 25, for use in combination with exercise therapy.

27. The cell preparation according to any one of claims 1 to 26, for use in a subject having inflammation in muscle tissue.

28. The cell preparation according to any one of claims 1 to 27, in form of intravenous administration.

29. The cell preparation according to any one of claims 1 to 28, wherein
the muscle mass loss is due to aging.

30. The cell preparation according to any one of claims 1 to 29, for administration to middle-aged or elderly people.

31. A cell preparation for use in treatment of age-related muscle loss, comprising:
the cell preparation for use in suppressing muscle mass loss according to any one of claims 1 to 30.

32. The cell preparation according to claim 31, wherein
the age-related muscle loss is sarcopenia or frailty.
